(19) Europäisches Patentamt
European Patent Office
Office européen des brevets

(11) EP 0 927 048 B1

(12) **EUROPEAN PATENT SPECIFICATION**

(45) Date of publication and mention of the grant of the patent:
**12.11.2003 Bulletin 2003/46**

(21) Application number: **97940263.3**

(22) Date of filing: **12.09.1997**

(51) Int Cl.7: **A61K 49/00**, G01R 33/58

(86) International application number:
**PCT/GB97/02509**

(87) International publication number:
**WO 98/010797 (19.03.1998 Gazette 1998/11)**

# (54) MARKER COMPOSITIONS

MARKERZUSAMMENSETZUNGEN

COMPOSITIONS DE MARQUEURS

(84) Designated Contracting States:
**AT BE CH DE DK ES FI FR GB IT LI NL SE**

(30) Priority: **12.09.1996 GB 9619118**
**30.05.1997 US 48051 P**

(43) Date of publication of application:
**07.07.1999 Bulletin 1999/27**

(73) Proprietor: **Amersham Health AS**
**0401 Oslo (NO)**

(72) Inventors:
- **BRILEY-SAEBOE, Karen Catherin**
  **0401 Oslo (NO)**
- **KELLAR, Kenneth Edmund**
  **Flemington, NJ 08822 (US)**
- **LADD, David Lee**
  **Wayne, PA 19087 (US)**
- **HOLLISTER, Kenneth Robert**
  **Chester Springs PA 19425 (US)**
- **BJOERNERUD, Atle**
  **N-0765 Oslo (NO)**

(74) Representative: **Canning, Lewis R.**
**Amersham plc**
**Amersham Place**
**Little Chalfont, Bucks. HP7 9NA (GB)**

(56) References cited:
**WO-A-94/09056**
**WO-A-95/26754**
**WO-A-96/40274**
**DE-A- 3 828 900**
**US-A- 4 888 555**
**US-A- 5 312 755**

- **WALKER P M ET AL: "A TEST MATERIAL FOR TISSUE CHARACTERISATION AND SYSTEM CALIBRATION IN MRI" PHYSICS IN MEDICINE AND BIOLOGY, vol. 34, no. 1, January 1989, pages 5-22, XP000006149 cited in the application**
- **BLECHINGER J C ET AL: "NMR PROPERTIES OF TISSUE-LIKE GEL MIXTURES FOR USE AS REFERENCE STANDARDS OR IN PHANTOMS" MEDICAL PHYSICS, vol. 12, no. 4, July 1985, page 516 XP000654003**
- **CHELLQUIST, ERIC M. ET AL: "Binding constant determination of WIN 22169, a novel polymeric ligand" J. PHARM. BIOMED. ANAL., VOL. 12, NO. 8, PAGE(S) 1015-22, August 1994, XP000576798**
- **UEDA M ET AL: "PROPERTIES OF CHELATE POLYMERS CONTAINING ETHYLENEDIAMINETETRAACETIC ACID STRUCTURE" KOBUNSHU RONBUNSHI, vol. 32, no. 4, 1975, pages 225-228, 287 - 292, XP000615386 & KOBUNSHU RONBUNSHI, ENG. ED., vol. 4, no. 4, 1975, pages 287-292,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 927 048 B1

## Description

FIELD OF THE INVENTION

This invention relates to compositions useful as markers, e.g. external markers or equipment markers, or as calibration standards for magnetic resonance (MR) imaging investigations and to calibration sets of such markers.

BACKGROUND TO THE INVENTION

Magnetic resonance imaging is a widely used diagnostic imaging modality in which, conventionally an image of the subject (patient) is produced by computer manipulation of the MR signals emitted from the subject following excitation of water proton magnetic resonance transitions while the subject is within the primary magnetic field of the MR imaging apparatus. The MR signals from the subject are dependent on the strength of the primary magnetic field as well as on the characteristic relaxation times $T_1$ and $T_2$ of the water protons, the relaxation times themselves being dependent upon factors such as chemical environment and temperature.

An external marker is an object, usually a tube containing an aqueous matrix doped with a paramagnetic substance, which is placed in the MR imaging instrument with the patient and allows for a calibration or normalization of the signal intensity (SI) within a region of interest. Normalization of the SI, relative to the external marker, allows for standardization of the SI obtained during multi-centre studies, evaluation of organ function (dynamic imaging) during contrast enhanced MR investigations, T1 mapping, tissue characterisation and evaluation. The external markers may be designed to give a specific signal intensity which is both field and temperature independent and which simulates the SI of specific issue types e.g. organs, fat, and various cancer types. The external markers may also be used for routine MR instrument quality control.

Because clincal MR units do not all operate at the same primary magnetic field strength and because the temperature of the markers will be between that of the subject and the ambient temperature within the imaging apparatus, there is a need for external markers which give signal intensities which are substantially invariant over the temperature and magnetic field variations encountered in clinical practice.

External markers, since they are used to calibrate images from body tissue, are generally designed to give tissue equivalent signal intensities, i.e. signal intensities of the same order of magnitude as those from at least part of the MR image of the patient.

Walker et al, in Physics in Medicine and Biology 34: 5-22 (1988) describe a tissue-equivalent test material comprising a polysaccharide gel (agarose gel) and a paramagnetic gadolinium compound. Both $GdCl_3$ and an EDTA chelate of gadolinium were studied and the agarose/GdEDTA combination was found to be superior. By mixing these in various combinations, i.e. by varying the $1/T_1$ and $1/T_2$ values for the compositions, Walker et al. were able to mimic the MR signals of various in vivo tissues. The signal strengths of the compositions however were not substantially invariant over the temperature and field strength variations encountered in clinical practice.

Tofts et al., in Magnetic Resonance Imaging 11: 125-133 (1993) described a similar tissue-equivalent material, this time comprising NiDTPA in agarose gel. This material had low temperature dependance at 21°C at low $T_1$ values and the authors categorically stated that the marker material should not contain gadolinium.

Howe, in Magnetic Resonance Imaging 6: 263-270 (1988) provided a solution to the problem of temperature variations of marker signal intensity over a relatively narrow temperature range of 20 to 30°C and for relatively low $T_1$ values (< 500ms) by doping a $Ni^{2+}$ containing agarose gel (which had low $1/T_1$ temperature dependence) with $Cu^{2+}$. Under the conditions investigated by Howe, the $T_1$ relaxivities of both $Cu^{2+}$ and $Ni^{2+}$ are temperature dependent but of opposite polarities. Howe did not however provide a solution to the problem of temperature dependence over a wider, more clinically relevant temperature range, or of magnetic field strength dependence.

To a large extent the problems of temperature and field strength dependence encountered with external markers arise from the temperature dependence of the $1/T_1$ value of the aqueous matrix material.

We have now found however that external markers whose $1/T_1$ and/or $1/T_2$ values are substantially temperature and field strength independent over the range of temperature and field strength variations encountered in clinical practice can be produced with a wide range of clinically relevant $1/T_1$ and/or $1/T_2$ values using a combination of paramagnetic materials, one of which has an essentially temperature independent $T_1$ relaxivity ($r_1$) and/or $1/T_2$ relaxivity ($r_2$) at the relevant temperatures and field strengths and a second of which has a $r_1$ and/or $r_2$ temperature dependence which is of opposite polarity to the $1/T_1$ (or $1/T_2$) temperature dependence of the aqueous matrix (ie. if $d(1/T_1)/dT$ for the pure matrix (i.e. the matrix in the absence of the paramagnetic materials) is negative then $dr_1/dT$ for the second paramagnetic material is positive) and a $r_1$ value which, in the relevant temperature and field strength ranges, is greater than that of the first, temperature invariant, paramagnetic material. For particularly high desired $1/T_1$ values for the marker, the second paramagnetic material may be omitted, while for particular low $1/T_1$ values the first paragmagnetic material may be omitted.

Thus viewed from one aspect the invention provides a set of aqueous marker compositions (for imaging use, most preferably not in free flowing liquid form unless when in ready to use form this is disposed in a substantially headspace-free chamber in a closed container) each composition having a selected $1/T_i$ value which is substantially invariant over an at least 10 C° temperature range (preferably 25 to 35°C, especially 20 to 30°C) between 15 and 40°C and preferably over an at least ±2% (eg. at least 0.1T) magnetic field strength range about a selected field strength value between 0.01 and 5T (preferably between 0.2 and 2.0T), and especially preferably over a field strength range of 0.2 to 2.0T, more preferably 0.01 to 5T or further, and comprising an aqueous matrix material having a non-zero $1/T_i$ temperature dependence within said temperature range with distributed therein a first paramagnetic material having a $T_i$ relaxivity which is substantially invariant within said range(s) and/or a second paramagnetic material having within said range(s) a $T_i$ relaxivity which has a non-zero temperature dependence of opposite polarity to the temperature dependence of $1/T_i$ of said matrix material and which, especially if the first and second paramagnetic materials contain the same paramagnetic metal, is greater than that of said first material, said set containing a plurality (i.e. at least two, preferably at least 3, desirably up to at least 10) of said compositions with different selected $1/T_i$ values, e.g. in the range 0.3 to 120.0 $s^{-1}$ (for example 0.5 or 0.6 to 20.0 sec, such as 0.7 to 3.5 $s^{-1}$, preferably encompassing at least the range 1.0 to 2.5 $s^{-1}$ for $1/T_1$ and for $1/T_2$ in the range 5.0 to 100 $sec^{-1}$, preferably encompassing at least the range 10 to 30 $sec^{-1}$), said set preferably comprising at least one said composition containing said second paramagnetic material and at least one said composition containing said first paragmagnetic material, and wherein i in $T_i$ is 1 or 2.

Thus the compositions may be $1/T_1$ markers, where i is 1 or 2 or $1/T_2$ markers where i is 2. Preferably however the paramagnetic materials will be such that both $1/T_1$ and $1/T_2$ for the compositions will be substantially invariant over the selected range(s).

The compositions will advantageously have substantially invariant $1/T_i$ values over both a temperature range and a magnetic field strength range: however compositions which are substantially temperature invariant over a range of magnetic field strengths (eg. as shown in Figure 3 of the accompanying drawings) are particularly valuable, especially for use as relaxation rate standards as discussed below.

While only first and second paramagnetic materials are referred to above, the compositions of the invention may if desired contain further paramagnetic materials, eg. two or more such first materials and/or two or more such second materials.

The compositions of the set of the invention may be packed in bulk form for filling into containers to be used as external markers. Alternatively, and preferably, they are pre-packed as a set of individual containers suitable for use as external markers. Such individual containers, or the compositions themselves, may also advantageously incorporate a radioopaque material (e.g. a heavy metal or heavy metal compound (such as lead, barium or bismuth or a compound thereof) or an iodinated material, e.g. a triiodophenyl compound such as the agents metrizoate, metrizamide, iohexol, iodixanol, iopentol, iopamidol and iotrolan) so that the markers may also be used in X-ray (e.g. CT) investigations or an echogenic material so that they may also be used in ultrasound investigations.

The individual containers for external marker compositions may be of any suitable material, e.g. nonferrous metal or more preferably plastics, and may be rigid or flexible. The containers may be of any appropriate size but desirably will be tubular with an internal diameter of between 10 and 50 mm (eg 10 to 20 or 15 to 50 mm, preferably 20 to 40 mm, especially 30 mm) and a length between 2 and 200 cm (eg 5 to 20 cm, conveniently 5, 10 or 20 cm). The appropriate size will depend upon their intended application, e.g. on the species or body portion under investigation. The containers will also preferably be colour coded according to the $1/T_i$ value of the compositions, and if appropriate the magnetic field strength for which that $1/T_i$ value applies.

The compositions may also be provided as part of items of medical equipment, e.g. catheters, or attached to or as parts of items of clothing or other articles which may be worn by the patient under investigation.

Viewed from a further aspect the invention also provides a method of MR imaging characterised in that the volume being imaged contains an external marker composition according to the invention, e.g. one or more marker compositions from a set according to the invention.

The signal from an external marker composition may also be used to estimate the concentration within a given body region of a contrast agent that has been administered to a patient.

Thus viewed from a yet further aspect the invention provides a method of estimating contrast agent concentration within a region of interest in a patient which method comprises comparing the detected MR signal intensity from said region with the detected signal from a marker composition according to the invention, and preferably also with the detected signal from a contrast agent free region (preferably the same region without contrast). For any such contrast agent, the relaxivity of which would be known, the resultant calculation of an estimated contrast agent concentration is straightforward.

Viewed from a yet further aspect the invention provides a method of calibration of an MR apparatus wherein detected MR signal strength is calibrated using an external marker composition according to the invention, e.g. one or more marker compositions from a set according to the invention.

In this aspect, calibration may typically involve arranging the apparatus to normalize image intensity according

to the detected intensity for the marker composition.

In this regard it may be noted that a major problem with relaxation rate measurement standards has been the temperature control of the magnets. Thus for example in an MR apparatus used for relaxation rate measurements one may be certain of the strength of the magnetic field to a high degree of accuracy (eg. 11.75T/500 MHz) but one is rarely certain of the temperature of the probe containing the standard. Conventional standards exhibit temperature dependence at such fields and use of marker compositions according to the invention for which $1/T_1$ and/or $1/T_2$ is substantially temperature invariant at such fields is highly advantageous. Thus such marker compositions can be used as calibration standards or quality control standards for $1/T_1$ and/or $1/T_2$ measurements for general NMR instrumentation and not just for MR imaging apparatus.

The invention allows production of compositions which give an MR signal intensity which is field and temperature independent. This concept may be used not just in external markers but also in the marking of medical equipment, in particular invasive equipment such as catheters, needles, etc. Thus such equipment may be marked with a marker composition that not only renders the equipment visualizable in an interventional MR investigation but which gives a signal of constant relative strength irrespective of its location within the patient (e.g. due to its temperature independence). (By relative is meant relative to a particular tissue or another marker etc.). This may be achieved in a relatively straightforward fashion, e.g. by placing a marker composition according to the invention in a sheath surrounding the equipment (e.g. a catheter) or in a central tube surrounded by a second tube so allowing fluid delivery through the intervening annular space.

Viewed from a yet further aspect therefore the invention provides a medical device, e.g. an invasive device such as a catheter, incorporating a marker composition according to the invention, e.g. one or more marker compositions from a set according to the invention.

The compositions of the invention preferably take any physical form that avoids motion artefacts. Thus, although they may be in free flowing particulate form, for imaging uses they are preferably not free flowing liquids at the temperatures at which they are to be used unless they are enclosed in a chamber which is substantially free of any headspace. If packaged in this way, however, aqueous solutions are preferred, especially if packed in breakage resistant containers. Other suitable physical forms include solid, semi-solid, gel, highly viscous (ie. stiff) liquid and particulate forms.

Simple liquid compositions, e.g. solutions or suspensions in water, can be used as marker compositions according to the invention. The term aqueous matrix material, as used herein, is thus defined as including water as well as materials such as aqueous gels.

The compositions may, as mentioned above, contain other components but the primary components are the aqueous matrix material which serves to provide the composition with its physical form, and the paramagnetic materials.

The aqueous matrix may conveniently be a hydrated hydrophilic polymer, e.g. a plastic polymer, or a gel, e.g. a polyacrylamide gel or a polysaccharide gel such as an alginate, agarose or agar gel. The quantity of matrix forming material in the composition is not especially critical. What is required is that the resulting matrix be sufficiently aqueous to provide an adequate MR signal having the appropriate relaxation times and preferably that it should not be a free flowing liquid. Generally, the minimum amount of matrix forming agent should preferably be used. In practice 2% by weight agar gels have been found to be suitable. In one preferred embodiment however, the matrix forming material will be one which does not have specialized water binding sites which result in water binding having lifetimes of the order of a few microseconds. For $1/T_2$ invariant compositions, polyacrylamide gels are preferred.

In one embodiment of the invention, the proton density of the compositions may be selected to correspond to that of a particular target tissue. Indeed it is one preferred embodiment to provide sets of compositions with selected constant $1/T_i$ values and different proton densities, e.g. corresponding to liver parenchyma or lesions.

Thus one objective of these markers is to match the signal intensity of particular animal or human tissues. For example, it may be desired to use a marker to mimic a lesion with a $T_1$ of 1000 milliseconds. However, it would not be adequate to simply use a marker which is an aqueous solution or gel with a $T_1$ of 1000 milliseconds, because the proton density of the markers may be significantly different than the proton density of the lesion. In general, the aqueous solution markers have a proton density of very nearly 100%. In fact, for present purposes, it can be regarded as 100% since the proton content due to the paramagnetic agents is negligible with respect to the proton content due to the water. Tissue, on the other hand, typically contains about 80% water, so for equivalent $T_1$ values, the signal intensity of tissue will be less than that of water in an amount dictated by the ratio of their respective water contents. One solution to this is to provide markers of differing water-proton content to match that of the desired tissue. The best method for accomplishing this is to use $D_2O$; $D_2O$ has no proton content, yet its properties are very similar to water as to make its presence have no significant influence on the relaxivities of the paramagnetic agents or on gel-formation properties, if markers are to have a gel matrix. For example, if one wanted to construct a gel with 80% water proton content, one would make a solution containing 80% protons and 20% deuterons. This would be done by combining $H_2O$ and $D_2O$ in the appropriate amounts, taking into account their different densities and different molecular weights, as well as the purity of the $D_2O$ source, so that the proton content is 80% of the total moles of protons and deuterons.

Of course, the reduction of proton content is not restricted to the substitution of $D_2O$ for $H_2O$; any material that does not contribute to the proton signal intensity, but is homogeneously distributed throughout the (at least partially) aqueous matrix will suffice. One non-limiting example includes water-soluble fluorocarbons.

The paramagnetic materials in the compositions of the invention are preferably chelate complexes of a paramagnetic metal ion (preferably gadolinium) with a polychelant (a polymeric chelant capable of multiple metallation). Linear polychelants, i.e. having repeat units of the structure

$$\{Ch\text{-}L\}_n$$

(where Ch is a chelating moiety, L is hydrophilic or hydrophobic organic linker moiety, and n is an integer) are preferred. For the first (temperature invariant) paramagnetic material, the linker moiety is preferably hydrophilic (e.g. an oxygen interrupted alkylene chain such as $((CRH)_mO)_p(CRH)_m$ where R is hydrogen or alkyl (e.g. $C_{1-4}$alkyl especially methyl), m is 2 or 3 and p is an integer, i.e. a linear or branched polyalkyleneoxy chain) while for the second paramagnetic material the linker moiety is preferably a hydrophobic moiety, e.g. a linear or branched $C_{4-30}$ alkylene chain, preferably a $C_{5-16}$ chain. The chelating moiety may be the residue of any appropriate chelating agent capable of binding the paramagnetic metal, e.g. a residue of DTPA, DTPA-BMA, EDTA, DOTA, DO3A, DPDP, etc., preferably DTPA. Examples of appropriate polychelants are given in WO94/09056, WO94/08629, WO95/26754 and WO96/40274 the disclosures of which are incorporated herein by reference.

Particularly suitable examples of the first and second paramagnetic materials are the gadolinium polychelate Compounds A and B described in the Examples below.

Gadolinium polychelants of the type discussed above have previously been described as being suitable for use as in vivo MR contrast agents. Their use, formulated in aqueous gels, as external markers has not previously been described.

Thus viewed from a further aspect the invention provides an aqueous composition having a selected $1/T_i$ value which is substantially invariant over an at least 10 C° temperature range (preferably 25 to 35°C, especially 20 to 30°C) between 15 and 40°C and preferably over an at least ± 2% (eg. 0.1T) magnetic field strength range about a selected field strength value between 0.01 and 5T (preferably between 0.2 and 2.0T) and comprising an aqueous gel of a hydrophilic synthetic or natural polymer having a non-zero $1/T_i$ dependence within said temperature range with distributed therein a first gadolinium polychelate having a $T_i$ relaxivity which is substantially invariant within said range (s) and/or a second gadolinium polychelate having within said ranges a $T_i$ relaxivity which is greater than that of said first polychelate and which has a non-zero temperature dependence of opposite polarity to the temperature dependence of $1/T_i$ of said aqueous gel, where i in $T_i$ is. 1 or 2.

Viewed from a yet further aspect the invention also provides an aqueous gel composition comprising a gel forming hydrophilic polymer and a gadolinium chelate of a polychelant having repeat units of formula

$$\{Ch\text{-}L\}_n$$

(where Ch is a chelating moiety, L is a hydrophilic or hydrophobic organic linker moiety and n is an integer).

The polychelants used according to the invention conveniently have molecular weights of 1 to 1000 kD, especially 4 to 100 kD and conveniently are capable of metallation by at least three and preferably up to at least 100 paramagnetic metal ions.

As an alternative to the gadolinium polychelates discussed above, the parmagnetic material used in marker compositions may be a composite material with restricted water exchange with the surrounding matrix, e.g. a paramagnetic-loaded porous particulate such as a zeolite or carbon cage (see for example WO93/08846 and WO93/15768 and the documents cited in the attached search reports, the contents all of which are incorporated herein by reference) or, more preferably, a paramagnetic loaded liposome (see for example WO96/11023 and the documents cited in the attached search reports, the contents all of which are incorporated herein by reference).

Besides paramagnetic loaded zeolites (e.g. Gadolite® or $GdCl_2$ in Y-zeolite) and paramagnetic loaded liposomes (n.b. the term liposome is used herein to relate to unilamellar and multilamellar particles), one may also use compartmentalized systems such as closed biological membranes (e.g. blood cells) loaded with paramagnetic material as well as protein particles (e.g. denatured albumin as in Albunex®).

Thus for a system of an aqueous gel containing liposomes, with the liposomes containing water and a paramagnetic agent, water molecules are free to exchange between the inside and the outside regions of the liposome. The amount of water contained inside the liposomes is small in comparison to the amount of water located outside of the liposomes, so the dominant contribution to the MR signal from the system is from the water on the outside of the liposomes. Consequently, the entity which is a liposome containing a paramagnetic agent can be regarded as a paramagnetic agent by the water molecules located outside the liposome. So the exchange of water molecules pertinent in this present case is the exchange of water molecules between the interior and exterior regions of the liposomes; this is analogous to inner-sphere relaxation. In contrast, the outer-sphere relaxation for this liposome case is the diffusion of water molecules on the exterior of the liposome diffusing in the region of the liposome. This outer-sphere contribution to $1/T_1$ may be negligible since the size of the liposomes is much larger than that of the individual paramagnetic agents inside the liposome, and the concentration of the paramagnetic agent inside the liposome is fairly low. Consequently, the value of $1/T_1$ for a marker containing liposomes is dominated by this analogous "inner-sphere" contribution. When the exchange of water molecules between the inner and outer regions of the liposome is sufficiently slow, the value of $1/T_1$ for the marker will be substantially independent of magnetic field strength. Furthermore, exchange of water molecules between the inner and outer regions of the liposome can be made to be slow and to have an exchange rate that decreases with decreasing temperature, so that the temperature dependence of $1/T_{1w}$, the relaxation rate of pure gel, can also be offset. As a result, a marker composition with a value of $1/T_1$ that is substantially independent of both magnetic field strength and temperature can be produced. Moreover the independence of magnetic field strength may be over a larger range (eg. 2.35 x $10^{-4}$T to 25T) than is possible with compositions comprising simple paramagnetic molecules dispersed in the aqueous matrix, with the independence range being extended at both lower and higher fields. Of course, liposomes are only one example of the compartmentalized aqueous systems that could function for the purpose of these markers. Also, the paramagnetic agent inside of the liposomes has no restriction on its identity: any paramagnetic agent can be used. This is because the outer-sphere contribution to the water molecules on the outside of the liposome is negligible. Therefore, all that matters is that the relaxation rate of the water protons inside the liposome ($1/T_{1int}$) is increased, and that the exchange rate is slow enough to make the $1/T_1$ of the marker independent of magnetic field strength. It does not matter what the temperature or magnetic field dependence of $1/T_{1int}$ is, because it can be offset by controlling the exchange rate of water molecules between the inside and outside the liposome, e.g. by altering the make-up or permeability of the liposome membrane.

As with the gadolinium polychelates mentioned above, while paramagnetic loaded zeolites and liposomes have been proposed as MR contrast media, their use, formulated as aqueous gels, as external markers has not previously been described.

Thus viewed from a further aspect the invention provides an aqueous composition having a selected $1/T_i$ value which is substantially invariant over an at least 10 C° temperature range (preferably 25 to 35°C, especially 20 to 30°C) between 15 and 40°C and preferably over an at least ± 2% (eg. 0.1T) magnetic field strength range about a selected field strength value between 0.01 and 5T (preferably between 0.2 and 2.0T), and preferably over a field strength range of 0.2 to 2.0T, more preferably 0.01 to 5T or further, and comprising an aqueous gel of a hydrophilic synthetic or natural polymer having a non-zero $1/T_i$ dependence within said temperature range with distributed therein a material containing water and a paramagnetic substance in a compartmentalized structure (e.g. a paramagnetic loaded zeolite or liposome) permitting diffusion of water between the aqueous gel and the compartment containing said paramagnetic substance, where i in $T_i$ is 1 or 2.

Compositions containing combinations of paramagnetic materials so balanced as to provide substantial temperature invariance of $1/T_i$ at ambient and physiological temperatures are novel and form a further aspect of this invention. Viewed from this aspect the invention provides an aqueous composition having a selected $1/T_i$ value which is substantially invariant over an at least 10 C° temperature range (preferably 25 to 35°C, especially 20 to 30°C) between 15 and 40°C and preferably over an at least ±2% (eg. at least 0.1T) magnetic field strength range about a selected field strength value between 0.01 and 5T (preferably between 0.2 and 2.0T), and especially preferably over a field strength range of 0.2 to 2.0T, more preferably 0.01 to 5T or further, and comprising an aqueous matrix material having a non-zero $1/T_i$ temperature dependence within said temperature range with distributed therein (a) a first paramagnetic material (eg a first gadolinium polychelate) having a $T_i$ relaxivity which is substantially invariant within said range(s) and (b) a second paramagnetic material (eg a second gadolinium polychelate) having within said range(s) a $T_i$ relaxivity which has a non-zero temperature dependence of opposite polarity to the temperature dependence of $1/T_i$ of said matrix material and which, especially if said first and second materials contain the same paramagnetic metal is greater than that of said first paramagnetic material; where i in $T_i$ is 1 or 2; said selected $1/T_i$ value preferably being in the range 0.3 to 120.0 $s^{-1}$ (for example 0.5 or 0.6 to 20.0 sec, such as 0.7 to 3.5 $s^{-1}$, preferably in the range 1.0 to 2.5 $s^{-1}$ for $1/T_1$ and for $1/T_2$ in the range 5.0 to 100 $sec^{-1}$, preferably in the range 10 to 30 $sec^{-1}$).

As mentioned above, all of the compositions of the invention may if desired contain one or more further paramagnetic materials.

Suitable paramagnetic materials for the compositions of the invention include water-soluble salts and chelates of transition metals, lanthanides and actinides, organic persistent free radicals, and the like. However chelates of paramagnetic metal ions (especially Gd, Mn and Fe (e.g. GdIII, MnII, FeIII)) such as those described above and those described in the patent literature as appropriate for use as MR contrast agents are preferred, e.g. Gd DTPA, MnDPDP, Gd DTPA.BMA, Gd DTPA.polylysine, etc.

While the concentrations of the paramagnetic materials in the marker compositions will depend upon the $1/T_1$

and/or $1/T_2$ value of the aqueous matrix, or the $r_1$ and/or $r_2$ values of the particular paramagnetic materials and on the desired $1/T_1$ and/or $1/T_2$ value for the composition, for gadolinium compounds concentrations will generally be up to 20 mM Gd (per litre water), e.g. up to 5.0, preferably up to 1.0, especially preferably up to 0.7 mM Gd for each such compound.

The compositions of the invention moreover may contain substantially water-insoluble particulate paramagnetic substance, e.g. gadolinium or manganese compounds, e.g. apatites or hydroxyapatites.

By substantially temperature or magnetic field strength invariant, it is meant that $1/T_i$ or the $T_i$ relaxivity should vary by no more than 5% over the selected temperature and/or magnetic field strength range. Preferably, substantially invariant materials or compositions will show such invariance over a field strength range of from 0.1 to 5T and a temperature range of at least 20 to 40°C.

While it is preferred that the $1/T_2$ values for the $1/T_1$ marker compositions and the $1/T_1$ values for the $1/T_2$ marker compositions should also be substantially temperature and field invariant, some variability is tolerable. Preferably $1/T_x$ (where $T_x=T_1$ when i=1 and $T_x=T_2$ when i=2) variation over a 10 C° range between 20 and 40°C (eg. 25 to 35°C) is less than 25%, particularly less than 20%, more particularly less than 15%, and especially preferably less than 10%, and most preferably less than 5% (percentages here being calculated as 100 (1- $V_1/V_2$) where $V_1$ and $V_2$ are the values being compared and $V_2 > V_1$).

BRIEF DESCRIPTION OF THE DRAWINGS

Figure 1 is an NMRD profile for Compound A;
Figure 2 is an NMRD profile for Compound B;
Figure 3 is a $1/T_1$ profile for compositions A to D according to the invention;
Figures 4 and 5 are NMRD profiles for pure 2% agar gel;
Figures 6A and 6B are NMRD profiles for a marker composition in 5% polyacrylamide gel and for the the gel alone;
Figure 7 is a $1/T_1$ profile for a marker composition in water;
Figures 8A and 8B are plots of signal enhancement (%ENH) as a function of time determined from studies of the pituitary gland;
Figure 9 is a plot of variation of signal intensity for markers placed on a grid within the body (phased array) coil of a 1.5T Siemens Vision mr imager; and
Figure 10 is a schematic representation in cross-section of a marker according to the invention.

The proton Larmor frequencies shown in the Figures correspond equally to field strengths. Thus 10 MHz is equivalent to 0.235T, 1 MHz to 0.0235T etc.

DETAILED DESCRIPTION OF THE INVENTION

To obtain a marker where $1/T_1$ and/or $1/T_2$ is independent of temperature, a matrix containing either a paramagnetic ion-containing substance having magnetic proton longitudinal and transverse relaxivities ($r_1$ and $r_2$, respectively) that are essentially independent of temperature, a paramagnetic ion-containing substance having temperature dependent magnetic longitudinal and transverse relaxivities ($r_1$ and $r_2$, respectively) that are of opposite temperature dependence to the relaxation rates of the pure aqueous matrix (ie. the matrix in the absence of the paramagnetic materials), or a combination of these, is made. The aqueous solid matrix can be for example a plastic polymer, but is preferably water (eg $H_2O$ or an $H_2O/D_2O$ mixture) or a gel, such as agar or agarose, particularly preferably water or a polyacrylamide gel. Whatever the substance used, the final matrix is preferably not in a free flowing liquid state, unless packed in a headspace free chamber so as to avoid any motion artifacts in the MR image, but must contain water so that the marker can give an appropriate MR signal. Preferred paramagnetic ion-containing substances are Gd-DTPA-polymeric conjugates such as Compounds A and B. The particular combination of paramagnetic ion-containing substances required will depend on the desired $1/T_1$ and/or $1/T_2$ of the marker and on the $1/T_1$ and/or $1/T_2$ value of the pure matrix to which no such substances have been added. The value of $1/T_i$ for the marker is a sum of the $1/T_i$ of the pure matrix, the $1/T_i$ value arising from the first paramagnetic ion-containing substance, and the $1/T_i$ value arising from the second paramagnetic ion-containing substance.

$1/T_1$ and $1/T_2$ for a pure aqueous matrix material are generally substantially independent of magnetic field strength above about 0.235T and generally reduces as temperature increases in the range 15 to 40°C.

By way of illustration, compositions with substantially invariant $1/T_1$ comprising an aqueous agar gel and the above-mentioned Gd-DTPA-polymeric conjugates (Compounds A and B) will be referred to in the following discussion. This is for illustrative purposes only, and is in no way intended to limit the scope of the present invention.

Compound A has a relaxivity that is essentially independent of temperature between 20°C and 40°C and of magnetic field strength above about 0.47 Tesla, while Compound B has a relaxivity that increases significantly with increasing temperature between 20°C and 40°C and is essentially independent of magnetic field strength above 0.47 Tesla. By using Compound A alone, Compound B alone, or combining the two in the appropriate quantities, markers having a particular $1/T_1$ value that is substantially independent of magnetic field strength and temperature can be produced.

Before proceeding with a description of the scientific principles involved in the invention, it is convenient to define $1/T_1$, $1/T_2$, $r_1$, and $r_2$, and to establish the relationships between them.

$1/T_1$ is the magnetic longitudinal relaxation rate of water protons in units of 1/seconds (1/s). Its reciprocal, $T_1$, is the longitudinal relaxation time (units of s).

$1/T_2$ is the magnetic transverse relaxation rate of water protons in units of 1/seconds. Its reciprocal, $T_2$, is the transverse relaxation time (units of s).

$r_1$ is the longitudinal relaxivity of a paramagnetic agent.

$r_2$ is the transverse relaxivity of a paramagnetic agent.

$r_1$ and $r_2$ are related to $1/T_1$ and $1/T_2$, respectively for Gd-containing paramagnetic agents, as follows:

$$r_i = \frac{\frac{1}{T_i} - \frac{1}{T_{iw}}}{[Gd]} \qquad i = 1,2 \tag{1}$$

where $1/T_{iw}$ is the longitudinal (i=1) and transverse (i=2) relaxation rate of a gel that contains no paramagnetic agent, and [Gd] is the millimolar (mM) concentration of gadolinium. The units for relaxivity are therefore $mM^{-1}s^{-1}$. Relaxivity may be considered the "relaxation efficacy" of the paramagnetic agent: the higher the relaxivity, the lower the concentration of agent that is required to attain a given enhancement in the relaxation rate ($1/T_i$) over that of the pure gel ($1/T_{iw}$).

Where a marker consists of more than one paramagnetic agent in an aqueous gel, Eq. 1 can be written in a more convenient form:

$$\frac{1}{T_i} = r_{ia}\,[Gd]_a + r_{ib}\,[Gd]_b + \frac{1}{T_{iw}} \qquad i = 1,2 \tag{2}$$

where $r_{ia}$ is the relaxivity of the first agent (Compound A), $r_{ib}$ is the relaxivity of the second agent (Compound B), $[Gd]_a$ is the concentration of Gd from the first agent, and $[Gd]_b$ is the concentration of Gd from the second agent.

It is important to note that $r_{ia}$ and $r_{ib}$ are generally dependent on temperature and magnetic field strength. It is an advantage to formulate the markers of the present invention with a paramagnetic ion-containing substance such as Compound A, which has longitudinal and transverse relaxivities that are independent of temperature and magnetic field strength. The quantity $1/T_{iw}$ is also dependent on temperature, but its dependence on magnetic field strength may be very minimal, especially for gels that have a low percentage of weight of gelling material to volume of the total gel. Consequently, the value of $1/T_i$ is dependent on temperature and magnetic field strength, and it is precisely this quantity that has been made independent of temperature and magnetic field strength in the markers of the present invention.

The quantities $1/T_{iw}$, $r_{ia}$, and $r_{ib}$ will now be examined independently, for obtaining a $1/T_i$ that is independent of temperature and magnetic field strength depends on an understanding of the temperature and magnetic field dependence for each of these three parameters. Also, for the components considered in the current invention, the value of $1/T_2$ is directly related to the value of $1/T_1$. That is, if the temperature and magnetic field dependence of $1/T_1$ is measured, that of $1/T_2$ can be calculated very accurately. Because it is much easier to measure the magnetic field dependence of $1/T_1$, this parameter will be considered alone to simplify the following discussion.

<u>The quantity $1/T_{1W}$</u>

For the conditions of the present invention, the value of $1/T_{1W}$ can be considered to be dependent on temperature and independent of magnetic field strength, at least at magnetic field strengths that are pertinent to most MRI conditions (about 0.0235 Tesla and higher). The temperature dependence of $1/T_{1W}$ is significant, particularly for lower values of $1/T_1$. For example, the values $1/T_{1W}$ of for a 2% (w/v) agar gel at clinically relevant temperatures are given below in Table 1.

Table 1

| Temperature (°C) | $1/T_{1W}(s^{-1})$ |
|---|---|
| 20 | 0.503 |
| 25 | 0.450 |
| 30 | 0.416 |
| 35 | 0.389 |
| 40 | 0.350 |

It is because of the temperature dependence of $1/T_{1W}$ that the presence of a paramagnetic ion-containing substance, or a combination of two paramagnetic ion-containing substances, is required to obtain a $1/T_1$ that is independent of temperature. Consequently, the quantities $r_{1a}$ and $r_{1b}$ must now be considered as they are of central importance in offsetting the temperature dependence of $1/T_{1W}$.

The quantities $r_{1a}$ and $r_{1b}$

For the quantity $1/T_{1W}$, it is generally sufficient to consider its temperature dependence. For the quantities $r_{1a}$ and $r_{1b}$, their dependence on magnetic field strength and temperature must be considered. Note that both $r_{1a}$ and $r_{1b}$ are independent of gadolinium concentration.

The quantity $r_{1a}$

The quantity $r_{1a}$ is the relaxivity of Compound A. Its temperature and magnetic field dependence are illustrated in Figure 1.

The plot displayed in Figure 1 is a nuclear magnetic relaxation dispersion (NMRD) profile. Two important characteristics of Compound A are displayed. First, the relaxivity above 10 MHz (corresponding to 0.235 Tesla) varies very little with magnetic field strength. Second, the relaxivity at all magnetic field strengths has very little temperature dependence; the temperature dependence is so slight that it is insignificant for the present purposes. However, as Eq. 2 shows, the fact that $r_{1a}$ is independent of temperature does not necessarily mean that $1/T_1$ will be independent of temperature (though it does mean that $1/T_1$ will be independent of magnetic field strength in the clinically relevant range). This is because of the temperature dependence of $1/T_{1W}$, which can make a significant contribution to $1/T_1$, making $1/T_1$ temperature dependent, especially for smaller values of $1/T_1$. For the case that $1/T_{1W}$ makes a significant contribution to $1/T_1$, it becomes necessary to add Compound B in addition to Compound A so that the temperature dependence of $1/T_{1W}$ can be offset.

The quantity $r_{1b}$

The quantity $r_{1b}$ corresponds to the relaxivity of Compound B. Its dependence on temperature and magnetic field strength is given in Figure 2.

In contrast to Compound A, the relaxivity of Compound B at all magnetic field strengths is dependent on temperature. This temperature dependence, an increase in relaxivity with increasing temperature, is exactly the behavior required to offset the temperature dependence of $1/T_{1W}$, which decreases with increasing temperature (see Table 1 above). Like the values of $r_{1a}$ and $1/T_{1W}$, the value of $r_{1b}$ has no significant dependence on magnetic field strength above 10 MHz (0.235T).

Composition of the markers

Using the results from Table 1 and Figs. 1 and 2 in Eq. 2, markers with $1/T_1$ values that are substantially independent of temperature and magnetic field strength can be constructed. Equation 2 is valid at any given value of temperature and magnetic field strength. Because the values of $r_{1a}$, $r_{1b}$, and $1/T_{1W}$ are independent of magnetic field strength above 10 MHz or 0.235 Tesla, the choice of magnetic field strength is arbitrary. However, at least two different temperatures must be chosen so that a set of simultaneous equations can be solved to obtain the proper amounts of compound A and compound B to be used. The simplest case is to set the value of $1/T_1$ at 35°C equal to that at 25°C, and solve these two equations simultaneously for $[Gd]_a$ and $[Gd]_b$. This simple case is very effective because the temperature of the marker may be expected to be between room temperature and the body temperature of the patient,

which should fall between 25°C and 35°C. Depending on the desired value of $1/T_1$ for the gel, it may not always be possible to use a combination of Compounds A and B; there are cases where only one of these agents can be used. Below, three cases are considered, corresponding to gels having three different values of $1/T_1$: an intermediate value where a combination of Compounds A and B is required; a large value, where only Compound A is required; and a small value, where only Compound B is required.

Intermediate values of $1/T_1$

An example of an intermediate value of $1/T_1$ can be taken as 1.7 $s^{-1}$. Taking relaxivities at 20 MHz, the following two equations result by using Eq. 2 at 35°C and 25°C for a 2% agarose gel.

$$1.7 = 6.305[Gd]_a + 9.901[Gd]_b + 0.389$$

(for 35°C, see Table 1)

$$1.7 = 6.109[Gd]_a + 8.730[Gd]_b + 0.450$$

(for 25°C, see Table 1)

Solving simultaneously for $[Gd]_a$ and $[Gd]_b$, the following result is obtained:

$$[Gd]_a = 0.171 \text{ mM (Compound A)}$$

$$[Gd]_b = 0.023 \text{ mM (Compound B)}$$

Therefore to make a gel with a $1/T_1$ value of 1.7 $s^{-1}$, the total gadolinium concentration is 0.194 mM, 0.171 mM arising from Compound A and 0.023 mM arising from Compound B. It is important to note that the concentration of gadolinium in mM pertains to the total volume of water that is present, and not to the total volume of the gel. To obtain accurate gadolinium concentrations, the volume fraction water, or the volume fraction of material other than water, must be known.

Large values of $1/T_1$

An example of a large value of $1/T_1$ is 3.3 $s^{-1}$. The simultaneous equations are:

$$3.30 = 6.305[Gd]_a + 9.901[Gd]_b + 0.389 \text{ (for 35°C)}$$

$$3.30 = 6.109[Gd]_a + 8.730[Gd]_b + 0.450 \text{ (for 25°C)}$$

with the solutions:

$$[Gd]_a = 0.515 \text{ mM (Compound A)}$$

$$[Gd]_b = -0.034 \text{ mM (Compound B)}$$

Note that a negative concentration of Compound B is indicated, which of course is an impossible situation. This solution has the physical interpretation that the paramagnetic contribution to temperature dependence of $1/T_1$, arising from Compound A ($r_{1a}[Gd]_a$ in Eq. 2) and Compound B ($r_{1b}[Gd]_b$ in Eq. 2), is greater and in opposite direction that the temperature dependence of the gel ($1/T_{1w}$). In other words, the temperature dependence of the gel ($1/T_{1w}$) has been overcompensated. Consequently, a negative concentration of Compound B would be required to make the paramagnetic contribution such that $1/T_1$ would be independent of temperature. There is no combination of Compounds

A and B that will not cause the temperature dependence of $1/T_{1w}$ to be overcompensated, so Compound A must be used alone.

The concentration of Compound A required for a $1/T_1$ of 3.30 $s^{-1}$ is calculated as follows. Using initially Eq. 2 for 35°C:

$$3.30 = 6305[Gd]_a + 0.389$$

from which:

$$[Gd]_a = 0.462 \text{ mM}$$

Now, to check if the temperature dependence of $1/T_1$ will be acceptable, its value is calculated for 25°C:

$$1/T_1 = 6.\ 109[0.462] + 0.450$$

$$1/T_1 = 3.27 \text{ s}^{-1}$$

Therefore, there is no significant temperature variation introduced by using Compound A by itself to obtain a total gadolinium concentration of 0.462 mM. A "significant" variation is defined as a greater than 5% variation between the two values.

<u>Small values of $1/T_1$</u>

An example of a small value of $1/T_1$ is 0.80 $s^{-1}$.

Proceeding as before:

$$0.80 = 6.305[Gd]_a + 9.901[Gd]_b + 0.389 \text{ (for 35°C)}$$

$$0.80 = 6.109[Gd]_a + 8.730[Gd]_b + 0.450 \text{ (for 25°C)}$$

the following solution is obtained:

$$[Gd]_a = -0.023 \text{ mM (Compound A)}$$

$$[Gd]_b = 0.056 \text{ mM (Compound B)}$$

In this case, a negative concentration of Compound A is required, which is not a possible situation. This situation is analogous to the second case above, where short values of $1/T_1$ are required, but now Compound B must be used by itself:

$$0.80 = 9.901\ [Gd]_b + 0.389$$

from which is obtained:

$$[Gd]_b = 0.042 \text{ mM}$$

Again, it must be checked that there is no significant variation in $1/T_1$ with temperature.

$$1/T_1 = 8.730[0.042] + 0.450$$

$$1/T_1 = 0.81\ s^{-1}$$

This temperature variation is also insignificant, and Compound B can be used by itself to obtain a total gadolinium concentration of 0.042 mM.

A More General Method for Obtaining the Composition of the Markers

There is a more general method for obtaining the composition of the markers that does not assume that $1/T_1$ or $1/T_{1w}$ is independent of magnetic field strength as was done in the preceding examples. The dependence of $r_{1a}$, $r_{1b}$, $1/T_1$, and $1/T_{1W}$ on temperature is still taken into account. For example, we can set up equations for $1/T_1$ at three different temperatures and magnetic field strengths:

At 20 MHz
$1/T_1 = 6.305[Gd]_a + 9.901[Gd]_b + (1/T_{1w})$ at 35°C
$1/T_1 = 6.309[Gd]_a + 9.316[Gd]_b + (1/T_{1w})$ at 30°C
$1/T_1 = 6.109[Gd]_a + 8.730[Gd]_b + (1/T_{1w})$ at 25°C

At 30 MHz
$1/T_1 = 6.122[Gd]_a + 9.815[Gd]_b + (1/T_{1w})$ at 35°C
$1/T_1 = 6.205[Gd]_a + 9.247[Gd]_b + (1/T_{1w})$ at 30°C
$1/T_1 = 6.011[Gd]_a + 8.590[Gd]_b + (1/T_{1w})$ at 25°C

At 50 MHz
$1/T_1 = 5.998[Gd]_a + 9.680[Gd]_b + (1/T_{1w})$ at 35°C
$1/T_1 = 6.125[Gd]_a + 9.193[Gd]_b + (1/T_{1w})$ at 30°C
$1/T_1 = 6.088[Gd]_a + 8.548[Gd]_b + (1/T_{1w})$ at 25°C

Note that $1/T_{1w}$ is no longer assumed to be independent of magnetic field strength. There are now nine equations and two unknowns, from which a "best-fit" to the two unknowns $[Gd]_a$ and $[Gd]_b$ can be obtained for a desired value of $1/T_1$. Note that as many equations as needed could be set up for as many temperatures and magnetic field strengths as required; a set of two unknowns and as many equations as needed would then be solved.

Thus, using the two different paramagnetic substances according to the invention unlike the compositions of Howe (supra) one can compensate for temperature variations in $1/T_{1w}$ over a clinically relevant wide temperature range and also produce markers having a clinically relevant wide range of $1/T_1$ values.

Moreover, while the $r_1$ relaxivities of the paramagnetic materials are each largely independent of magnetic field strength above 0.235T, the paramagnetic materials and the aqueous matrix can be so combined as to give a marker composition which is essentially field independent at even lower fields, e.g. down to 0.0235T.

There are thus three main conditions necessary for the composition of the paramagnetic ingredients of the present invention.

1. One ingredient (for example Compound A) must have a relaxivity that is essentially independent of temperature;

2. One ingredient (for example Compound B) must have a relaxivity that changes with increasing temperature in a fashion opposite to the change in increasing temperature of $1/T_{1w}$. Furthermore, if the second ingredient contains the same paramagnetic metal as the first, its relaxivity should be higher than that of the first (temperature independent) ingredient so that the temperature variance of the background relaxation rate of pure gel ($1/T_{1w}$) can be offset; and

3. Both ingredients should have a relaxivity that varies very little, if at all, with magnetic field strength above 0.235 Tesla, and preferably as low as 0.01 Tesla.

It can be appreciated that all three of these conditions have one important principle in common, which is the chemical exchange of a water molecule (or water protons) between the inner coordination sphere of the paramagnetic ion and the bulk must be reasonably slow, with the bulk being all of the water molecules (or water protons) that are

present in the marker but not bound in the inner coordination sphere of a paramagnetic ion.

Slow water exchange is difficult to define quantitatively. For example, [Gd(DTPA-BMA)($H_2O$)] is considered to have a slower exchange rate than many other Gd-containing chelates, and specific values of the exchange rate and the activation parameters (which describe the temperature dependence of the exchange rate) are given by Gonzalez et al. in J. Phys. Chem. 98: 53-59 (1994). There are, however, two contributions to the total relaxivity, the inner sphere and outer sphere contributions (see Koenig et al., Progress in NMR Spectroscopy 22: 487-567 (1990)). The inner sphere contribution arises from the exchange of water molecules between the inner coordination sphere of the paramagnetic ion and the bulk. The outer sphere contribution arises from diffusion of water molecules in the vicinity of the paramagnetic agent. Consequently, slow water exchange only influences the magnitude of inner sphere contribution.

The main influence of slow water exchange on the inner sphere contribution is to cause it to be smaller than would be the case if the water exchange were more rapid. Also, the water exchange rate becomes slower as the temperature is decreased, causing the inner sphere contribution to decrease with decreasing temperature. The decrease in the magnitude of the inner sphere contribution with decreasing temperature is opposite to that of the outer sphere mechanism, which has important consequences for the present invention. When the decrease in the magnitude of the inner sphere contribution is matched by the increase in magnitude of the outer sphere contribution as the temperature decreases at a particular magnetic field strength, the two contributions offset each other and the relaxivity is independent of temperature. This is the case for Compound A.

Creating a paramagnetic agent like Compound B that has a relaxivity that decreases with decreasing temperature requires that the decrease in the magnitude of the inner sphere contribution be greater than the increase in the magnitude of the outer sphere contribution. For this reason, where the paramagnetic metals used are the same, a second agent with a higher relaxivity than Compound A must be chosen. Polymeric agents with relaxivities that are higher than Compound A, yet which still have slow water exchange, are achieved by increasing the magnitude of the inner sphere contribution, since the outer sphere contribution is expected to be about the same for all polymeric Gd-containing agents. Compound B is such an agent, with the higher relaxivities resulting from the presence of intramolecular hydrophobic interactions (see Kellar et al., Proc. Int. Soc. MR in Medicine, 4th Scientific Meeting and Exhibition, New York, NY, 1996, p. 75). It is important that Compound B has relaxivities that are higher than Compound A because that is the only way that the decrease in relaxivity with decreasing temperature can be large enough to offset the increase in $1/T_{1w}$ of the gel with decreasing temperature.

Slow water exchange also gives the added benefit that it causes the relaxivities to have a smaller variance with magnetic field strength than would be the case for more rapid water exchange. This is illustrated by Koenig (in Investigative Radiology, 29(Suppl.): S127- S130 (1994)), but it may be noted that the decrease in relaxivity above 0.235 Tesla can be avoided by using smaller macromolecules than considered in the reference, as evidenced by the relaxivities shown for Compounds A and B in Figures 1 and 2, respectively.

The quantity $1/T_{2W}$

As disclosed previously, for most conditions of the present invention, the value of $1/T_{1W}$ can be considered to be dependent on temperature but independent of field strength. The same can be said for $1/T_{2W}$. Although $1/T_{1W}$ and $1/T_{2W}$ are similar in magnitude for many systems, it is not a generality. This is illustrated by agarose gels, where the $1/T_{2W}$ can be much greater than $1/T_{1W}$. The reason for this is similar to the reason that $1/T_2$ is much greater than the value of $1/T_1$ in tissue and in cross-linked BSA samples (Koenig et al., Magn. Reson. Med. 29: 77-83 (1993)), and is described in detail in K.E. Kellar, S.H. Koenig, K. Briley-Sæbø, M. Spiller, Agarose Gels as a Simulated Tissue Matrix for MRI: The Importance of 3-D Gel Structure and Magnetization Transfer, Proceedings of the International Society for Magnetic Resonance in Medicine, Fifth Scientific Meeting and Exhibition, Vancouver, B.C., Canada, April 12-18, 1997, p. 1567, the disclosure of which is hereby incorporated by reference. The basic problem is that, even in the presence of paramagnetic agents, $1/T_{2W}$ can be so large that it will dominate $1/T_2$. Consequently, because $1/T_{2W}$ is temperature dependent, markers containing the appropriate paramagnetic agents cannot be made to be independent of temperature unless the value of $1/T_{2W}$ for the marker is very large, which restricts the range of possible $1/T_{2W}$ values for the markers. The data in Example 8 below for 2% agarose gel illustrates this problem - even at the highest $1/T_2$ (19.05 $s^{-1}$), $1/T_{2W}$ accounts for 82% of the this value.

The unique properties of the present markers make them useful for a wide variety of applications, including use as a standardization or positional reference in MR imaging of a subject, the standardization or calibration of MR hardware, mapping field inhomogenieties of coils, and standardization of results obtained from studies conducted in different locations or at different times at the same location.

The signal-to-noise (SNR) ratio is currently the most important quality assurance (QA) parameter within MRI in order to evaluate instrument performance as well as to quantitate relative changes in signal intensity. The SNR ratio is determined as the ratio of the signal intensity with in a region of interest (ROI) divided by the standard deviation of the noise (MRI in the Body, Charles Higgins, et al., Raven press, 1992). When obtaining SNR ratios, the ROI within

the tissue or object imaged should contain at least 100 pixels. The signal intensity within the ROI is the mean value of the pixel intensity within the ROI minus any offset. The standard deviation of the noise is determined from an ROI which is a non-signal producing background area. SNR ratios are often used to evalauate the changes in signal intensity within a region of interest after the administration of a contrast agent. The relative difference in the SNR ratios pre- and post-contrast is described by the percent enhancement (% ENH). It is possible to determine clearance of the contrast agent by monitoring signal intensity changes (through changes in % ENH), since the signal intensity is related to the concentration of contrast agent. However, as previously noted, the absolute signal intensity is obtained relative to the standard deviation of the noise. Since the standard deviation of the noise can be affected by many factors, most importantly scaling factors, it is not very reliable for quantitation purposes. Endogenous markers, such as fat or specific tissue, are not reliable due to their inhomogenous and variable signal intensities.

The markers of the present invention, however, give a region of constant signal intensity irrespective of changes in scaling factors, position with respect to distance away from the patient (temperature indenpendence), and the strength of the magnetic field. They can therefore be used to normalize the signal intensity by calculating the "signal-to-marker" (SMR) ratio. This is determined as the ratio of the signal within an ROI of the tissue, divided by the signal intensity within a given region of the marker. Since the SMR ratio is not affected by many of the variables effecting the SNR ratio, the use of the markers to normalize signal intensities should consequently be superior to using the standard deviation of the noise. The following studies verify these claims.

Standardization of signal intensity is another application of these markers. When comparing images obtained at different imaging centers, variations among the different instruments such as the operating field strength, noise levels, and coils (body, head, phased-array, etc.), make it difficult to directly compare signal intensities obtained within a specific region of interest. By using the SMR ratio, these variations are minimized and enable direct and quantitative comparison of data among the centers. Such a comparison is shown below in Example 10. The patients included in these studies had pathology, and some variation in the %ENH betwen patients should be expected. However, for the %ENH to be meaningful, variation in its value must only be reflective of patient-to-patient variability and not due to differences in instrumentation. When obtained with SMR, the possible %ENH values range from 22 to 38; with SNR, from 1 to 51. Therefore, estimation of contrast agent uptake is very difficult when obtained using SNR. These results suggest another advantage of using the SMR; it may be possible to enroll a smaller number of patients in clinical trials as a result of the lower standard deviations in %ENH.

The results of Example 10 below show that calculating %ENH with respect to SMR is superior to calculating %ENH with respect to SNR. A reasonable hypothesis would be that a marker with a signal intensity that varies field strength, such as one containing a given amount of GdDTPA, would be sufficient to obtain a SMR. However, the standard deviation of the signal intensity of such a marker would also lead to errors in the calculation of %ENH, just as the VSDN does. Consequently, if the standard deviation of the signal intensity of the markers would have a contribution from the markers themselves, this would lead to errors in the calculation of %ENH. One such error would be if the signal intensity of the markers varied with temperature, and would result if the distance the marker is placed from the patient is not the same for both pre- and post-contrast measurements. By way of illustration, the signal intensity of GdDTPA varies about 20% at MRI fields between temperatures of 20°C to 35°C (essentially room temperature to body temperature), as based on relaxivity data (K.E. Kellar et al. Magn. Reson. Med. 37: 730-735 (1997)). This variance would be expected for most metal chelates (it would be slightly less for GdDTPA-BMA, which has a relaxivity that does not vary as strongly with temperature). The markers of the current invention have signal intensities which are not dependent on temperature, which is an important advantage. Another important advantage is that the signal intensities of the present invention do not vary significantly with field strength at field strengths pertinent to MRI, which enables direct %ENH comparisons obtained from instruments operating at different field strengths. This is because the $T_1$ values of the markers do not vary with field strength, as demonstrated in the previous NMRD profiles. However, the highest field used for NMRD is only 1.2 T, while much MR imaging is done at 1.5 T. The data shown in Example 11 below, which compares $T_1$ values obtained at 0.47 T on a Bruker Minispec and $T_1$ values obtained at 1.5 T on a MRI instrument, shows that the $T_1$ of the markers are independent of field strength up to 1.5 T.

The uptake and clearance rate of a contrast agent from a tissue, as measured by the changes in %ENH, can give critical information related to the function or pathology of the tissue. Examples include, but are not limited to, the characterization of breast lesions by monitoring the rate of uptake and rate of washout of gadolinium chelates, and the evaluation of tissue viability in the brain and heart by monitoring the rate of perfusion of a contrast agent within the tissue. It is important to note that all applications which utilize changes in signal intensity to classify or characterize tissue function should be greatly improved by using the SMR ratios instead of traditional SNR ratios. This is shown in Example 12 below. When using the %ENH from the SMR ratios it is possible to obtain clearance values (half-lives) of the contrast agent in a given tissue; this is not possible when using the SNR data due to the clearance curve being not well-defined. Also, the signal should theoretically return to baseline once the contrast agent is cleared from the tissue. A related use of the markers would be to ensure that the proper dosage of contrast agent was administered; certain signal intensities are expected for certain dosages, and the signal intensities are easily checked relative to

those of the markers.

The markers of the present invention may also be used to validate and control imaging hardware. One application is the evaluation of rf-intensity variations within MRI coils. The rf maps can be used to identify areas of inhomogeneous rf energy that cause variations in the observed signal intensities. This information can be useful in identifying areas of high variation (usually close to the coil surface), as well as giving an indication of the magnitude of the variance.

As disclosed above, the markers of the present invention can be formulated in a wide variety of matrices. Water is a preferred matrix material, as are gels such as agarose and polyacrylamide. When selecting a matrix material to contain the paramagnetic agents, it is important to take into consideration components or characteristics of the matrix material that may alter the relaxivity of the agents, thereby compromising the temperature and magnetic field insensitivity of the markers. For example, while agarose gel is solid macroscopically, microscopically the gel contains water with a mobility that is not different enough from pure water to affect the relaxivity of the paramagnetic agents. Consequently, markers comprised of agarose gel will have $T_1$ values with the desired magnetic field and temperature independence. On the other hand, if a "gel-like" matrix were made of sucrose, for example, the viscosity of water from the point of view of the paramagnetic agent would be significantly increased. Because the rate of rotational motion of the paramagnetic agent is related to the viscosity of the water (as sensed by the paramagnetic agent), and the rate of rotational motion affects the relaxivity of the paramagnetic agent, the relaxivity of the paramagnetic agent increases with increasing viscosity. Therefore, it becomes difficult to make markers unless the relaxivity of the paramagnetic agents is measured under the exact conditions of the matrix material where a viscous (as sensed by the paramagnetic agent) environment results. This can become tedious, and other factors such as the. solid fraction of the matrix (ie. the volume of the matrix material that is not water), must also be considered. Although it is possible to make markers under these difficult conditions, it is not practical, and therefore it is preferable to use a matrix material that has a micro-environment (the environment sensed by the paramagnetic agents) similar to pure water, for example agarose or polyacrylamide gels. However, as noted above, a gel that does not have an aqueous micro-environment that is significantly different from pure water as to affect the relaxivity of the paramagnetic agent(s), can be used. Whereas agarose and polyacrylamide are most preferred, preferred gels with a microenvironment not significantly different from pure water include those made from bovine serum albumin, human serum albumin, alginate, cellulose, starch, polyvinyl alcohol, and various gums.

A marker formulation in a gel-type matrix is preferred if there is some concern about the contents coming into contact with a patient if the container were to break. However, for simple calibration and control applications, water is preferred. If a liquid matrix is used, the marker formulation will preferably fill substantially the entire volume of the container, so as to minimize motion artifacts. Practically any type of container can be used to hold the marker formulation, as long as the materials of which the container is made are non-MR active. The choice of container type and size is dictated by the intended application. For example, the marker formulation can be contained within a flexible container of any size or shape, but is preferably cylindrical, such as tubing made of plastic, rubber, polypropylene, and the like, allowing the markers to follow the shape of the patient. The flexible container may be of any size, depending on the area to be imaged; for example, the tube can be of sufficient length to surround the head or a body extremity such as a leg or arm, or to be draped across a region of the trunk such as the pelvic, abdominal, or thoracic region. The inner diameter of the flexible container should preferably be at least approximately the "voxel" (volume pixel) size of the display screen of MRI equipment, currently about 0.5 mm, and can be as large as necessary to permit proper positioning of the marker on the subject's body. The preferred size of the inner diameter of the flexible container is 1.5 cm to 5 cm, most preferred is from 2 cm to 4 cm, and particulary preferred is an inner diameter of 3 cm. The container may also be made of any rigid non-MR active material including plastic, polycarbonate, glass, and the like, or any combination thereof. Glass vials with plastic stoppers are an example of such a container. Such rigid containers can be any shape and size, the dimensions of the container being dictated by the intended application. A preferred container is generally cylindrical, and is is 10 cm long and 3 cm in diameter. A particulary preferred size is 5 cm long and 3 cm in diameter. Markers made with these types of containers are useful in calibrating or standardizing MRI hardware or for imaging areas of the body where the marker can be placed near, adjacent to, or on the body at a discrete location (such as for the head or extremities, the armpit, crook of the elbow or knee, etc.). The containers holding the marker formulation could also be placed within or secured in a removable fashion to a garment or article which can be worn by or draped over the patient, including stockings, brassieres, caps, gloves, belts, head-, arm-, or wrist-bands, hats or caps, and blankets. The containers may also be releasably secured to a subject's body or to an object to be placed with the MRI magnet, for example with string or by adhesive tape, velcro, glue, etc. Containers may also be placed within or secured to an object in a removable fashion, as long as the object is made of non-MR active material, so that the object containing the marker can be carried into the magnet during an examination or imaging session. It is anticipated that such an object containing a marker, preferably a toy or stuffed animal, can be used in procedures involving children. Carrying a toy or stuffed animal into the magnet will provide comfort to the child during the procedure, while at the same time allowing a marker to be placed within the magnetic field. The marker may then be removed from the toy after the procedure, and the toy can be given to the child. The use of such toys in an imaging procedure forms a

further aspect of the invention.

One example of a marker is shown schematically in Figure 10 of the accompanying drawings. Referring to Figure 10, a sealed cylindrical container 1 (for example a polycarbonate container) contains an aqueous marker composition 2, eg an agarose or polyacrylamide gel containing one or more paramagnetic compounds in solution. Preferably there is no headspace, ie gas-pocket, within the chamber in the container in which the marker composition is disposed; however if the composition is not in free-flowing liquid form, eg where it is in gel form, a headspace may be tolerable.

The invention will now be illustrated by the following non-limiting Examples.

## Example 1

Preparation of Compound A

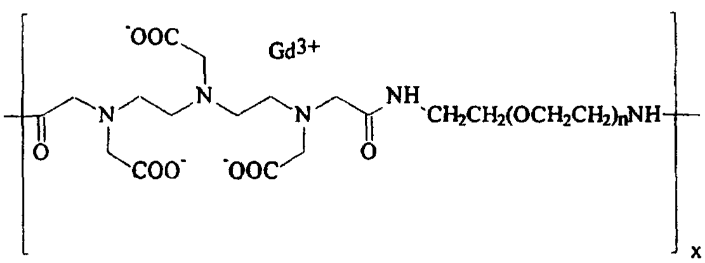

A solution of 1.12 g (0.856 mmol) of bis-tosylate prepared from polyethyleneoxide of average MW 1450 in 22 mls. of absolute ethanol was cooled in an ice bath and a stream of ammonia was introduced over a period of 25 minutes. The reaction mixture was heated in a stainless steel reactor at 100°C for 16 hours, then cooled to room temperature and filtered. The filtrate was concentrated to remove solvent, treated with water (22 mls) and 1.0 N NaOH (3.4 mls) and extracted twice with $CHCl_3$. The $CHCl_3$ extracts were dried over anhydrous magnesium sulfate and concentrated to leave 0.77 g of bis-amine.

A solution of 0.66 g (0.66 mmol) of the bis-amine in 3.3 mls of DMSO was treated with triethylamine (0.184 mls, 1.32 mmol) and a solution of 0.236 g (0.66 mmol) of diethylenetriaminepentaacetic acid dianhydride in DMSO (3.3 mls). The reaction mixture was stirred at room temperature for 1 hour, then treated with 26 mls of water. The resultant solution was filtered through a 0.45 micron filter and the filtrate diafiltered against water in a diafiltration cell equipped with a 5000 MW cut-off membrane leaving 24 mls of solution which was treated with a two fold excess of gadolinium (III) chloride hexahydrate. This solution was diafiltered against water employing a 5000 MW cut-off membrane leaving the title product. Average Mol. Wt. 20.2 kD (measured by size exclusion chromatographic HPLC against PEG standards). Gd content 7.09% by weight (by ICP analysis).

The NMRD profile of Compound A is shown in Figure 1.

**Example 2**

Preparation of Compound B

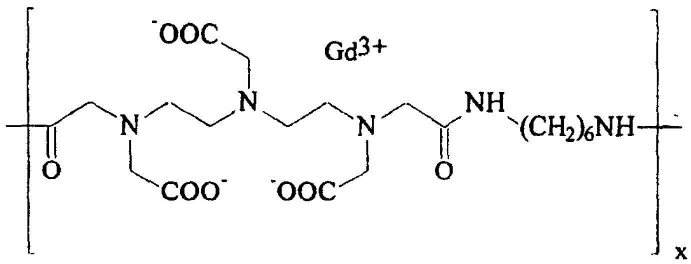

To a solution of 2.97 g (25.5 mmol) of 1,6-hexanediamine in 45.1 ml of dimethylsulfoxide were added 11.08 ml (79.5 mmol) of triethylamine and 9.45 g (31.8 mmol) of diethylenetriaminepentaacetic acid dianhydride with vigorous stirring. The resulting reaction mixture was stirred at ambient temperature for 28 hours to give a homogeneous solution, following which it was diluted to approximately 1% solids content with water and diafiltered for 5 turnovers using a nominal 10,000 MW cut-off, spiral wound, polysufone diafiltration membrane. The resulting aqueous retentate was then freeze-dried to yield a hygroscopic white solid.

15.0 g of this solid was dissolved in 600 mls of deionized water and stirred at moderate speed as it was slowly treated with a 5% aqueous solution of gadolinium (III) chloride hexahydrate. The addition was continued until a small test sample, dripped into PAR test reagent, caused a color change from pale yellow to deep yellow. The PAR test reagent had been prepared previously by sonicating a mixture of 40 mls of deionized water, 20 mls of trace metal grade ammonium hydroxide, and 0.005 g of 4-(2-pyridylazo)resorcinol (from Aldrich Chem. Co., CAS Registry Number 61-25-6) for one minute. Following sonication, it was treated with 5.7 mls of trace metal grade acetic acid, allowed to cool to ambient temperature, and diluted to 100.0 mls with additional deionized water.

Upon observing the color change in the PAR reagent, the polymer complex was diafiltered as before for 6 turnovers, following which the pH was adjusted to 6.5 with 3.0 M NaOH, giving the title product. The title product was then freeze-dried to produce a fluffy white solid. Average Mol. Wt. 16.7 kD (measured by size exclusion chromatographic HPLC against PEG standards). Gd content 21.46% by weight (by ICP analysis).

The NMRD profile of Compound B is shown in Figure 2.

**Example 3**

Preparation of Gadolinium Polymer Markers in 2% agar gel

Preparation of 2% (wt/wt) agarose gel

1.00 g of granulated purified Agar-Agar (Merk KGaA) was weighed into five 50 ml glass vials. 0.075 g of Sodium benzoate (Fluka Chemika-BioChemika) and 0.075 g of potassium sorbate (>99%, Fluka Chemika-BioChemika) were weighed into each of the five samples. 50.0 mL of purified water was pipetted into each of the five samples. The samples were sealed with Teflon clamp tops and placed in a boiling water bath for 20 minutes until a clear solution was observed. The samples were removed from the water bath and allowed to cool to 60-70°C prior to the addition of the gadolinium complexes.

NMRD profiles of the pure gel are shown in Figures 4 and 5.

Preparation of the gadolinium polymers

Table 2 shows the amount of Compounds A and B required to prepare the external markers with $1/T_1$ ($s^{-1}$) values of 1.15, 1.31, 1.68 and 3.03 $s^{-1}$. One gel blank was also prepared.

Table 2:

| Required amounts of Compounds A and B. | | | | |
|---|---|---|---|---|
| Sample | Amount (mL) of Compound B [Gd]=24.9 mM | Amount (mL) of Compound A [Gd]=10.7 mM | Total sample volume (mL) | Total[Gd] (mM) |
| A | 0.145 | 0.0 | 50.145 | 0.072 |
| B | 0.133 | 0.285 | 50.398 | 0.1163 |
| C | 0.081 | 0.710 | 50.791 | 0.1893 |
| D | 0.0 | 2.240 | 52.240 | 0.4588 |

Samples were prepared from solutions of Compounds A and B with total gadolinium concentrations of 10.7 and 24.9 mM Gd respectively. (Note all gadolinium concentrations were determined by ICP analysis).

The required amounts of Compounds A and B were pipetted into a warm agar sample. The sample was gently homogenized for one minute. 2.0 mL of the warm gel was pipetted into three NMR tubes. The tubes were capped and immediately placed in an ice bath for five minutes until a solid gel was formed.

Relaxation analysis

All samples were analyzed at 0.47T using a BRUKER Pc120b Minispec equipped with a circulator bath able to maintain probe head temperatures of 15 to 50°C. All longitudinal relaxation rates were measured at 20, 25, 30, 35 and 40°C using an inversion recovery sequence. The relaxation rates were calculated from a mono-exponential three parameter fit of the signal amplitude of 20 data points versus time. The transverse relaxation rates were measured at 20, 25, 30, 35 and 40°C using a CPMG spin echo sequence. The echo amplitude of every second echo was obtained using an echo time of 4 ms. The relaxation rates were calculated from a three parameter fit of echo amplitude versus time.

Figure 3 shows the results of $1/T_1$ measurements performed on the samples on a field-cycling relaxometer located at New York Medical College, Valhalla, New York. This instrument has been thoroughly described in Koenig et al., Progress in NMR Spectroscopy 22: 487-567 (1990), which is herein incorporated by reference.

Referring to Figure 3, it should be noted that the variance with field strength (Proton Larmor Frequency) in the range 1 to 10 MHz (0.0235 to 0.235T) arises from $1/T_{1w}$ rather than from field dependency of $r_{1a}$ or $r_{1b}$.

The values of $1/T_1$ for each of the markers do not change significantly above 20 MHz as a function of temperature or magnetic field strength. In this range, these markers are adequate for the purposes of the current invention. However, although their temperature dependence is very small, there is a slight decrease in the $1/T_1$ values between 1 MHz and 20 MHz. This decrease is due to the contribution of $1/T_{1w}$ from the agar gel to the overall $1/T_1$, which is not independent of magnetic field strength as previously anticipated. Consequently, this problem that is due to the field dependence of $1/T_{1w}$ can be significantly reduced, if not eliminated, by using a gel other than those made of agarose.

It may be noted that the curvature in the NMRD profiles of the pure gel, meaning the decrease in $1/T_{1w}$ with increasing magnetic field strength, is similar to that of $1/T_1$ of the markers. This shows that for markers made with agar gels, the decrease $1/T_1$ in with increasing magnetic field strength is caused by the agar gel. The dependence on magnetic field strength of the agar gel is shown in Figure 5.

The NMRD profiles are similar to those oberved in tissue (see Koenig et al., Progress in NMR Spectroscopy 22: 487-567 (1990) and Koenig et al., Mag. Res. Medicine 29: 77-83 (1993)), and therefore water protons must relax by a similar mechanism. In particular, some of the water molecules involved in hydration of the gel must have "lifetimes", or how long a water molecule is bound to the gel, on the order of a few microseconds. These water molecules are bound in specialized binding sites, ostensibly bound by three or four hydrogen bonds. Consequently, to create markers with $1/T_1$ values that are independent of magnetic field strength from 0.01 MHz (0.0235 Tesla) and higher, a gel should be used that does not have these specialized water binding sites. This will also reduce undesirable magnetic field dependency of the $1/T_2$ values for the markers.

**Example 4**

Preparation of Markers in Water

A stock solution of Compound A in distilled water with a total Gd concentration of 4.02 mM, and a stock

solution of Compound B in distilled water with a total Gd concentration of 7.03 mM, were prepared. The relative concentrations of the two stock solutions to be used in a marker with a given projected $T_1$ were determined as described previously. A total volume of 10 mL was used for each marker; the volume used for each of the stock solutions is listed below.

| Projected $T_1$(ms) | Volume of Compound A Stock Added (mL) | Volume of Compound B Stock Added (mL) |
|---|---|---|
| 1000 | 0.0746 | 0.0755 |
| 800 | 0.200 | 0.0646 |
| 600 | 0.409 | 0.0515 |
| 400 | 0.828 | 0.0215 |

In order for the markers to have clinical utility, the markers must give stable $T_1$ results as a function of sample storage. The shelf-life stability of the marker solutions were evaluated by analyzing the longitudinal relaxaiton times over a nine month storage period. The samples were stored at 4 °C during the storage period. The results of the stability study are summarized in Table 3 below.

Table 3:

| Shelf-life stability of marker solutions prepared in water. | | | |
|---|---|---|---|
| Sample Number | Initial Analysis | 4 Month Analysis | 9 Month Analysis |
| | $T_1 \pm$ sd (ms) | $T_1 \pm$ sd (ms) | $T_1 \pm$ sd (ms) |
| 1 | 432 ± 4 | 427 ± 6 | 427 ± 5 |
| 2 | 638 ± 6 | 638 ± 8 | N.A. |
| 3 | 828 ± 9 | 824 ± 9 | 819 ± 11 |
| 4 | 1033 ± 11 | 1036 ± 13 | 1027 ± 17 |

Samples were stored at 4 °C. SD represents the standard deviaiton of three sample replicates analysed at 25 °C and three sample replicates analyzed at 35 °C (n=6). The results of the stability study indicate the markers will have shelf-life stability for adequate clinical utility.

**Example 5**

Polyacrylamide gels

Polyacrylamide gels are commonly used in electrophoresis of proteins (Gel electrophoresis of proteins: A practical approach, 2 ed, B.D. Hames et al, 1990). Gels at 4% (wt/v) and 5% (wt/v) were prepared from an acrylamide-bisacrylamide stock solution containing 30% (wt/v) acrylamide and 0.8% (wt/v) bisacrylamide (Bio-Rad). Markers at four different $T_1$ values were prepared using stock solutions of polymeric contrast Gd chelate in distilled water with the following gadolinium concentrations: Compound B [Gd] = 24.9 mM and Compound A [Gd] = 10.7 mM . The samples were prepared as shown in Table 4 below.

Table 4:

| Preparation of external markers in 4% and 5% polyacrylamide gel. | | | | | | | | |
|---|---|---|---|---|---|---|---|---|
| % | Sample Number | 30:0.8 polyacrylamide (ml) | 0.5 M sodium phosphate (ml) | 1.5% ammonium persulphate (ml) | RO water (ml) | TEMED (ml) | CmpndB (ml) | CmpndA (ml) |
| 4 | 1 | 60 | 31.5 | 2.25 | 359.8 | 0.45 | 0.94 | 0.05 |
| | 2 | 60 | 31.5 | 2.25 | 353.7 | 0.45 | 1.22 | 0.90 |
| | 3 | 60 | 31.5 | 2.25 | 350.8 | 0.45 | 1.21 | 3.80 |
| | 4 | 60 | 31.5 | 2.25 | 345.0 | 0.45 | 1.18 | 9.60 |

Table 4: (continued)

| Preparation of external markers in 4% and 5% polyacrylamide gel. | | | | | | | | |
|---|---|---|---|---|---|---|---|---|
| % | Sample Number | 30:0.8 polyacrylamide (ml) | 0.5 M sodium phosphate (ml) | 1.5% ammonium persulphate (ml) | RO water (ml) | TEMED (ml) | Cmpnd B (ml) | Cmpnd A (ml) |
| 5 | 1 | 8.305 | 3.50 | 0.250 | 37.8 | 0.05 | 0.100 | 0.00 |
| | 2 | 8.305 | 3.50 | 0.250 | 37.0 | 0.05 | 0.133 | 0.080 |
| | 3 | 8.305 | 3.50 | 0.250 | 37.3 | 0.05 | 0.116 | 0.455 |
| | 4 | 8.305 | 3.50 | 0.250 | 35.9 | 0.05 | 0.050 | 1.954 |

The required concentrations of Compound B and Compound A were calculated using the method previously described. The relaxation rates of the background gels as a function of temperature are shown in Table 5 below.

Table 5:

| Background relaxation rates of polyacrylamide gels. | | |
|---|---|---|
| Gel % | R1 ($s^{-1}$), 20°C | R1 ($s^{-1}$), 35°C |
| 4 | 0.626959 | 0.487115 |
| 5 | 0.621813 | 0.493949 |

**Example 6**

$T_1$ and $T_2$ values of external markers in 4% and 5% polyacrylamide gels

The longitudinal and transverse relaxation times of the samples of Example 5 were analyzed at 0.47 T using a Bruker Minispec as function of temperature. The results are shown in Table 6 below.

Table 6:

| Longitudinal relaxation times of markers prepared in polyacrylamide gels as a function of temperature. | | | | | |
|---|---|---|---|---|---|
| Gel % | Sample Number | T1 ± sd* (ms) 20 C | T1 ± sd (ms) 25 C | T1 ± sd (ms) 30 C | T1 ± sd (ms) 35 C |
| 4 | 1 | 1212 ± 5 | 1217 ± 3 | 1206 ± 2 | 1211 ± 4 |
| | 2 | 923 ± 4 | 905 ± 1 | 889 ± 1 | 892 ± 5 |
| | 3 | 661 ± 2 | 643 ± 4 | 636 ± 2 | 638 ± 4 |
| | 4 | 416 ± 2 | 412 ± 2 | 402 ± 2 | 408 ± 4 |
| 5 | 1 | 923 ± 2 | 934 ± 5 | 939 ± 5 | 960 ± 6 |
| | 2 | 767 ± 4 | 765 ± 5 | 765 ± 2 | 764 ± 3 |
| | 3 | 567 ± 2 | 565 ± 2 | 566 ± 3 | 573 ± 3 |
| | 4 | 283 ± 2 | 280 ± 2 | 280 ± 1 | 281 ± 1 |

*sd = the standard deviation of a three replicate analysis.

An NMRD profile was obtained for the markers prepared in 5% polyacrylamide. The results which show the relaxation rates as a function of field strength and temperature (20 °C to 35 °C) are shown in Figure 6A (Figure 6B shows the NMRD profile of the 5% polyacrylamide gel alone). Note that the the variance of $1/T_1$ with temperature and field strength, although small, is greater than that of the markers prepared in agar gel (Fig. 3) and in water (Fig. 7). This is due to the significant dispersion of $1/T_{1W}$ with field strength in the region most pertinent to MRI (field strengths corresponding to those above 10 MHz). For agarose gels, the dispersion has occured below 1 MHz, so the values of $1/T_{1W}$ have much less dependence on field strength. For water, the dispersion does not occur until field strengths higher than those practical for MRI, and most NMR, applications are attained, so the value of $1/T_{1W}$ is independent of

field strength for present purposes. Consequently, it is expected that markers prepared in water will have the most preferred characteristics (temperature and field insensitive values of $1/T_1$).

**Example 7**

Influence of variatons in the standard deviation of the noise

Clinical studies, involving 52 patients and three centers were performed in order compare the utility of the SMR ratio relative to the SNR ratio. Table 7 summarizes the number of patients used.

Table 7:

| Clinical studies performed in order to evalaute the clinical utility of the external markers. | | | | |
|---|---|---|---|---|
| Study Number | Number of patients | Intrument type | Field Strength | Pulse Sequences |
| 1 | 16 | Siemens magnet, phased array coil | 1.0 T: Expert | GE, $T_1$-W |
| 2 | 30 | Siemens magnet, body coil | 1.5 T: Vision | GE, $T_1$-W |
| 3 | 6 | Phillips magnet, body coil | 1.5 T | SE, $T_1$-W, TR/TE=450/18 |

Both studies 1 and 2 were performed in order to evaluate the uptake of a Mn-containing contrast agent in the liver. The patients in this study had liver pathology and images were obtained pre-injection and within four hours post-injection. Study 3 investigated the changes of signal intensity in the pituitary gland over a three month time period after the injection of a Mn-containing contrast agent.

Table 8 shows data from ten patients in studies 1 and 2. The percent enhancement (% ENH) was calculated according to equation 3.

$$\%ENH = 100 - \left( \frac{SR_{pre}}{SR_{post}} \times 100 \right)$$

where SR is either the SNR ratio or the SMR ratio.

Table 8:

| Effect of variance in the standard deviation of the noise on the %ENH. | | | | | |
|---|---|---|---|---|---|
| Study Number | Patient | %ENH based on SNR | variance of the SD of the noise, (%) | %ENH based on **SMR** | variance of the SD of the SI of marker, (%) |
| 2 | 5 | -8.57 | 28.4 | 41.55 | 8.9 |
| 2 | 11 | 68.16 | 51.9 | 41.80 | 4.9 |
| 2 | 19 | -2.62 | 29.0 | 31.30 | -0.6 |
| 2 | 21 | 8.06 | 10.8 | 24.78 | 1.1 |
| 2 | 27 | 40.67 | 0.0 | 40.95 | 0.3 |
| 1 | 2 | 16.51 | 13.6 | 27.65 | 0.0 |
| 1 | 6 | 26.51 | 0.0 | 27.01 | -0.7 |
| 1 | 12 | 3.28 | 28.6 | 30.86 | 0.1 |
| 1 | 13 | 2.64 | 20.0 | 22.11 | 0.0 |
| 1 | 17 | -37.41 | 52.2 | 34.7 | -0.6 |

The results from Table 8 clearly show that the variance of the standard deviation (VSDN) of the noise is critical in obtaining accurate %ENH. The variance of the VSDN is expressed as the percent variation in the standard deviation of the noise on images obtained prior to contrast and after contrast (on the same day). When the VSDN is large, inaccurate %ENH values are obtained. For example, a negative %ENH is impossible for conditions of the present study. One image from patient 19 in study 2, pre- and post-contrast, showed a clear visual increase in intensity after contrast administration; consequently, the negative %ENH obtained using the SNR is erroneous. On the other hand, the %ENH obtained by using SMR represents a reasonable assessment of its value. Furthermore, when the VSDN is zero (patients 27 and 6 in studies 2 and 1 respectively), the %ENH obtained by SNR and SMR are equivalent, demonstrating that variations in the noise are the dominant source of errors in determination of %ENH.

**Example 8**

Preparation of external markers in 2% agarose gel

Agarose gel is commonly used as a suspension medium in NMR and also in the construction of phantoms. These gels are preferred since a stiff gel is formed, yet the diffusion coefficient of water in agarose is not significantly different from that in water. Marker solutions with varying $T_1$ values were prepared and evaluated in 2% (wt/v) agarose gels (Bacto-Agar, DIFCO) and the longitudinal and transverse relaxation rates were determined as a function of temperature at 0.47 T. The samples were prepared according the method disclosed previously in a total volume of 50 ml. The stock solutions of Compound B and Compound A had gadolinium concentrations of 24.9 mM Gd and 10.7 mM Gd, respectively. The results are shown in Table 9 below.

Table 9:

| Longitudinal and transverse relaxation rates of markers prepared in 2% agarose gels as a function of temperature at 0.47 T | | | | | | | | |
|---|---|---|---|---|---|---|---|---|
| Sample Number | Cmpnd B (ml) | Cmpnd A (ml) | R1 ± SD (ms) 20°C | R1 ± SD (ms) 25°C | R1 ± SD (ms) 30°C | R1 ± SD (ms) 35°C | R2 ± SD (ms) 20°C | R2 ± SD (ms) 35°C |
| 1 | 0.145 | 0.00 | 1.13 | 1.16 | 1.16 | 1.16 | 14.86 | 16.69 |
| 2 | 0.113 | 0.285 | 1.30 | 1.32 | 1.33 | 1.32 | 15.75 | 18.10 |
| 3 | 0.081 | 0.710 | 1.68 | 1.70 | 1.69 | 1.63 | 15.53 | 18.61 |
| 4 | 0.00 | 2.24 | 3.05 | 3.09 | 3.05 | 3.02 | 16.36 | 19.05 |
| Blank | 0.00 | 0.00 | 0.50 | 0.45 | 0.42 | 0.39 | 13.63 | 15.57 |

**Example 9**

Effect of agar concentration on the longitudinal and transverse relaxation rates of the marker formulation (R1 in water 1.66 $s^{-1}$)

The percentage of agarose in the gel could be decreased in order to obtain a smaller $1/T_{2W}$, which is demonstrated in Table 10. The effect of agarose gel concentration on the observed longitudinal and transverse relaxation rates was evaluated by preparing five samples with varying agarose gel concentration (0 - 1.98% (wt/v)). Only one marker formulation was evaluated (with R1 value 1.66 $s^{-1}$ in water). Samples were prepared by weighing agarose powder into a 50 ml glass vial. 49.209 ml of RO (reverse osmosis) water was then added to each sample and the vials were sealed. The samples were placed in boiling water bath for 20 minutes until a clear yellow solution was formed. The samples were removed from the water bath and 81 µl of a 24.9 mM Gd stock solution of Compound B and 710 µl of a 10.7 mM Gd stock solution of Compound A were added. 2.0 ml of each sample was pipetted into an NMR tube and the samples stored on ice for 30 minutes prior to analysis. The longitudinal relaxation rates were determined at 0.47 T and 40 °C.

Table 10:

| Effect of agar concentration on the longitudinal and transverse relaxation rates of the marker formulation (R1 in water 1.66 $s^{-1}$). | | |
|---|---|---|
| % Gel (wt/v) | R1 ($s^{-1}$) | R2 ($s^{-1}$) |
| 0.0 | 1.66 | 1.83 |
| 0.49 | 1.66 | 5.79 |
| 0.98 | 1.61 | 9.53 |
| 1.48 | 1.63 | 13.44 |
| 1.98 | 1.63 | 18.47 |

These results clearly show that if it is desirable to have markers where the value of $1/T_2$ must be independent of temperature and field strength, it is best to use a gel composed of a material other than agarose (such as polyacrylamide). However, in some cases it may be desirable to have $1/T_2$ values which are similar to a specific tissue. The marker can be modelled to simulate tissue by determining the required agarose concentration which gives the desired $1/T_2$ value. Although the use of agarose gels as a tissue phantom substance has been well described in the literature, the use of the marker formulation as a doping agent may have advantages over other proposed methods since $1/T_1$ of these gels will not be sensitive to temperature or field strength.

**Example 10**

Standardization of the SI

46 patients were imaged at two different hospitals (studies 1 and 2, see Example 7 above). The clinical studies were performed over a 4 month time period. The %ENH was calculated in the liver for each patient within 4 hours post injection of a contrast agent. The average %ENH values obtained from each center are presented in Table 11 below.

Table 11:

| %ENH in the liver after the administration of a contrast agent. SD represents the standard deviation of the %ENH values obtained within a patient group. | | |
|---|---|---|
| Study | SMR Average %ENH ± SD | SNR Average %ENH ± SD |
| 1 (n=16) | 29 ± 4 | 21 ± 20 |
| 2 (n=30) | 30 ± 8 | 30 ± 21 |

These results show the usefulness of the markers when comparing quantitative data between imaging centers.

**Example 11**

Estimation of $T_1$ by MRI

Table 12 below shows the $T_1$ values of marker formulations analyzed on a Bruker Minispec at 0.47 T and a clinial imager at 1.5 T. $SD_A$ reflects the standard deviation of nine sample replicates over a temperature range of 20°C to 40°C. $SD_B$ reflects the standard deviation of two replicate analysis.

Table 12:

| $T_1$ values of marker formulations | | | |
|---|---|---|---|
| Sample Number (as Example 4) | 0.47 T, Bruker Minispec | 1.5 T, Phillips System | % variation |
| | $T_1 \pm SD_A$ (ms) | $T_1 \pm SD_B$ (ms) | |
| 1 | 432 ± 4 | 449 ± 13 | -3.94 |
| 2 | 638 ± 6 | 664 ± 7 | -4.07 |
| 3 | 828 ± 9 | 864 ± 5 | -4.35 |

Table 12: (continued)

| $T_1$ values of marker formulations | | | |
|---|---|---|---|
| Sample Number (as Example 4) | 0.47 T, Bruker Minispec | 1.5 T, Phillips System | % variation |
| | $T_1 \pm SD_A$ (ms) | $T_1 \pm SD_B$ (ms) | |
| 4 | 1033 ± 11 | 1078 ± 9 | -4.36 |

Since all of the % variation (expressed relative to the 0.47 T values) are less than 5%, the $T_1$ values of the markers can be considered to be independent of magnetic field strength. This also shows that the markers can be used to calibrate software used by MRI instrumentation to obtain $T_1$ values.

**Example 12**

Uptake and Clearance Rates of Contrast Agent

The signal intensity of the pituitary gland in six patients was monitored over a 3 month time period after the administration of a contrast agent (study 3, see Example 7 above). The signal intensity within the gland was measured prior to injection, 2 to 4 hours post injection, and 1, 2, 3, 4, 8, and 12 weeks post injection. The percent enhancement (%ENH) was determined using equation 3 (see Example 7). The results are shown in Figure 8A and B. These results show that the clearance of the contrast agent in the pituitary gland is best characterized when the SMR ratio is used to calculate the %ENH (Fig. 8B). The large significant negative value obtained when using the SNR ratios does not represent the actual change in signal intensity of the tissue, but instead reflects the variations in the noise which are instrument, and not tissue, related.

**Example 13**

Quality Assurance of Imaging Hardware

The utility of rf-mapping was illustrated by positioning 43 markers within a phased-array coil. Only one marker formulation ($T_1$ in water of 618 ms) was used in the evaluation of the coil. The markers were prepared in 2.5 ml glass vials which were taped on a Plexiglass® grid and positioned inside the body (phased array) coil. Images were obtained on a 1.5 T Siemens Vision using a Turbo Spin Echo Sequence (TSE). In order to evaluate the ability of the Siemens compensation program to adjust for rf variations in the coil, images were obtained with and without the application of the compensation program. The results are shown in Figure 9. In Figure 9, the samples with highest signal intensity are the samples located closest to the coil. Samples 35 to 41 (ie Positions 35 to 41) represent samples in the isocenter of the coil. The large variations in the signal intensity are related to the variations in the rf field relative to position from the isocenter. The purpose of the compensation program is to improve the homogeneity within the coil, and this improvement is quantified by the use of the markers. The variation in signal intensity, represented as the relative standard deviation (%RSD) between the 43 samples, was determined for the phased array coil. Without compensation, the %RSD of the signal intensity was 45%, whereas after the application of the compensation program the %RSD of the signal intensity was only 20%.

## Claims

**1.** A set of aqueous marker compositions each composition having a selected $1/T_i$ value which is substantially invariant over an at least 10 C° temperature range between 15 and 40°C and comprising an aqueous matrix material having a non-zero $1/T_i$ temperature dependence within said temperature range with distributed therein a first paramagnetic material having a $T_i$ relaxivity which is substantially invariant within said range and/or a second paramagnetic material having within said range a $T_i$ relaxivity which has a non-zero temperature dependence of opposite polarity to the temperature dependence of $1/T_i$ of said matrix material, and which, if said first and second materials contain the same paramagnetic metal is greater than that of said first material, said set containing a plurality of said compositions with different selected $1/T_i$ values, comprising at least one said composition containing said second paramagnetic material and at least one said composition containing said first paramagnetic material, where i in $T_i$ is 1 or 2.

**2.** A set as claimed in claim 1 wherein each composition has a said selected $1/T_i$ value which is substantially invariant over the range 25 to 35°C.

**3.** A set as claimed in either of claims 1 and 2 wherein each composition has a said selected $1/T_i$ value which is substantially invariant over an at least ± 2% magnetic field strength range about a selected field strength value between 0.01 and 5T.

**4.** A set as claimed in any one of claims 1 to 3 containing a plurality of said compositions with different selected $1/T_i$ values encompassing the range 1.0 to $2.5s^{-1}$.

**5.** A set as claimed in any one of claims 1 to 4 containing at least one said composition containing said first paramagnetic material and at least one said composition containing said second paramagnetic material.

**6.** A set as claimed in any one of claims 1 to 5 containing at least one said composition wherein said aqueous matrix material is water.

**7.** A set as claimed in claim 6 wherein said water comprises $H_20$ and $D_2O$.

**8.** A set as claimed in any one of claims 1 to 7 containing at least one said composition wherein said aqueous matrix material is an aqueous gel.

**9.** A set as claimed in claim 8 containing at least one said composition wherein said gel is a polyacrylamide or agarose gel.

**10.** A set as claimed in any one of claims 1 to 9 wherein said compositions are disposed within closed containers.

**11.** A set as claimed in claim 10 containing at least one said composition which is disposed in a substantially headspace-free chamber in a said closed container and contains water as said aqueous matrix material.

**12.** An aqueous composition having a selected $1/T_i$ value which is substantially invariant over an at least 10 C° temperature range between 15 and 40°C and comprising an aqueous gel of a hydrophilic synthetic or natural polymer having a non-zero $1/T_i$ dependence within said temperature range with distributed therein (a) a first gadolinium polychelate having a $T_i$ relaxivity which is substantially invariant within said range and/or (b) a second gadolinium polychelate having within said range a $T_i$ relaxivity which is greater than that of said first polychelate and which has a non-zero temperature dependence of opposite polarity to the temperature dependence of $1/T_i$ of said aqueous gel, where i in $T_i$ is 1 or 2.

**13.** An aqueous gel composition as claimed in claim 12 comprising a gel forming hydrophilic polymer and a gadolinium chelate of a polychelant having repeat units of formula

$$\{Ch\text{-}L\}_n$$

(where Ch is a chelating moiety, L is a hydrophilic or hydrophobic organic linker moiety and n is an integer).

**14.** An aqueous composition having a selected $1/T_i$ value which is substantially invariant over an at least 10 C° temperature range between 15 and 40°C comprising an aqueous gel of a hydrophilic synthetic or natural polymer having a non-zero $1/T_i$ dependence within said temperature range with distributed therein a material containing water and a paramagnetic substance in a compartmentalized structure permitting diffusion of water between the aqueous gel and the compartment containing said paramagnetic substance, where i in $T_i$ is 1 or 2.

**15.** An aqueous composition having a selected $1/T_i$ value which is substantially invariant over an at least 10 C° temperature range between 15 and 40°C and comprising an aqueous matrix material having a non-zero $1/T_i$ temperature dependence within said temperature range with distributed therein (a) a first paramagnetic material having a $T_i$ relaxivity which is substantially invariant within said range and (b) a second paramagnetic material having within said range a $T_i$ relaxivity which has a non-zero temperature dependence of opposite polarity to the temperature dependence of $1/T_i$ of said matrix material and which, if said first and second materials contain the same paramagnetic metal is greater than that of said first paramagnetic material, where i in $T_i$ is 1 or 2.

**16.** A composition as claimed in claim 15 wherein said aqueous matrix material is water.

**17.** A medical device incorporating a marker composition as defined in any one of claims 1 to 16.

**18.** An article of clothing incorporating a marker composition as defined in any one of claims 1 to 16.

**19.** A method of MR imaging **characterised in that** the volume being imaged contains as an external marker a composition as defined in any one of claims 1 to 16.

**20.** A method as claimed in claim 19 wherein the imaging subject is human and said composition is disposed in a container releasably associated with a toy.

**21.** A method of calibration of a MR apparatus wherein detected MR signal strength is calibrated using a composition as defined in any one of claims 1 to 16.

**22.** A method of estimating contrast agent concentration within a region of interest in a patient which method comprises comparing the detected MR signal intensity from said region with the detected signal from a marker composition as defined in any one of claims 1 to 16.

**23.** A method as claimed in claim 22 wherein percent enhancement (%ENH) values are calculated from the signal-to-marker ratio (SMR).

**Patentansprüche**

**1.** Satz wässriger Markerzusammensetzungen, wobei jede Zusammensetzung einen gewählten $1/T_i$-Wert besitzt, welcher über einen mindestens 10°C-Temperaturbereich zwischen 15 und 40°C im wesentlichen invariant ist und umfassend ein wässriges Matrixmaterial mit einer Nicht-Null-$1/T_i$-Temperaturabhängigkeit innerhalb des Temperaturbereichs, worin darin verteilt sind ein erstes paramagnetisches Material mit einer $T_i$-Relaxivität, welche innerhalb dieses Bereichs im wesentlichen invariant ist, und/oder ein zweites paramagnetisches Material mit einer $T_i$-Relaxivität innerhalb dieses Bereichs, die eine Nicht-Null-Temperaturabhängigkeit entgegengesetzter Polarität zu der Temperaturabhängigkeit von $1/T_i$ des Matrixmaterials besitzt, und welche, wenn das erste und zweite Material das gleiche paramagnetische Metall enthalten, größer ist als diejenige des ersten Materials, wobei der Satz eine Vielzahl der Zusammensetzungen mit unterschiedlich gewählten $1/T_i$-Werten enthält, umfassend mindestens eine der Zusammensetzung, die das zweite paramagnetische Material enthält, und mindestens eine der Zusammensetzung, die das erste paramagnetische Material enthält, worin i in $T_i$ 1 oder 2 ist.

**2.** Satz nach Anspruch 1, wobei jede Zusammensetzung den gewählten $1/T_i$-Wert besitzt, welcher über den Bereich von 25 bis 35°C im wesentlichen invariant ist.

**3.** Satz nach Anspruch 1 und/oder 2, wobei jede Zusammensetzung den gewählten $1/T_1$-Wert besitzt, welcher über einen mindestens ± 2% Magnetfeldstärkebereich bei einem gewählten Feldstärkewert zwischen 0,01 und 5 T im wesentlichen invariant ist.

**4.** Satz nach mindestens einem der Ansprüche 1 bis 3, enthaltend eine Vielzahl der Zusammensetzungen mit unterschiedlich gewählten $1/T_i$-Werten, welche den Bereich von 1,0 bis 2,5 $s^{-1}$ umfassen.

**5.** Satz nach mindestens einem der Ansprüche 1 bis 4, enthaltend mindestens eine der Zusammensetzung, enthaltend das erste paramagnetische Material, und mindestens eine der Zusammensetzung, enthaltend das zweite paramagnetische Material.

**6.** Satz nach mindestens einem der Ansprüche 1 bis 5, enthaltend mindestens eine der Zusammensetzung, wobei das wässrige Matrixmaterial Wasser ist.

**7.** Satz nach Anspruch 6, wobei das Wasser $H_2O$ und $D_2O$ umfasst.

**8.** Satz nach mindestens einem der Ansprüche 1 bis 7, enthaltend mindestens eine der Zusammensetzung, wobei das wässrige Matrixmaterial ein wässriges Gel ist.

**9.** Satz nach Anspruch 8, enthaltend mindestens eine der Zusammensetzung, wobei das Gel ein Polyacrylamid- oder Agarosegel ist.

**10.** Satz nach mindestens einem der Ansprüche 1 bis 9, wobei die Zusammensetzungen innerhalb geschlossenen Behältern angeordnet sind.

**11.** Satz nach Anspruch 10, enthaltend mindestens eine der Zusammensetzung, die in einer im wesentlichen kopfraumfreien Kammer in dem geschlossenen Behälter angeordnet ist und Wasser als das wässrige Matrixmaterial enthält.

**12.** Wässrige Zusammensetzung mit einem gewählten $1/T_i$-Wert, der im wesentlichen über einen mindestens 10°C-Temperaturbereich zwischen 15 und 40°C invariant ist und umfassend ein wässriges Gel eines hydrophilen synthetischen oder natürlichen Polymeren mit einer Nicht-Null-$1/T_i$-Abhängigkeit innerhalb des Temperaturbereichs, worin darin verteilt sind (a) ein erstes Gadolinium-Polychelat mit einer $T_i$-Relaxivität, die innerhalb des Bereichs im wesentlichen invariant ist, und/oder (b) ein zweites Gadolinium-Polychelat, das innerhalb des Bereichs eine $T_i$-Relaxivität besitzt, welche größer ist als diejenige des ersten Polychelats und die eine Nicht-Null-Temperaturabhängigkeit entgegengesetzter Polarität zu der Temperaturabhängigkeit von $1/T_i$ des wässrigen Gels besitzt, worin i in $T_i$ 1 oder 2 ist.

**13.** Wässrige Gelzusammensetzung nach Anspruch 12, umfassend ein gelbildendes hydrophiles Polymer und ein Gadoliniumchelat eines Polychelatbildners mit Wiederholungseinheiten der Formel

$$[\text{-Ch-L-}]_n$$

(worin Ch eine chelatbildende Einheit ist, L eine hydrophile oder hydrophobe organische Linkereinheit ist, und n eine ganze Zahl ist).

**14.** Wässrige Zusammensetzung mit einem gewählten $1/T_i$-Wert, der über einen mindestens 10°C-Temperaturbereich zwischen 15 und 40°C im wesentlichen invariant ist, umfassend ein wässriges Gel eines hydrophilen synthetischen oder natürlichen Polymeren mit einer Nicht-Null-$1/T_i$-Abhängigkeit innerhalb des Temperaturbereichs, worin darin verteilt sind ein Material, enthaltend Wasser und eine paramagnetische Substanz in einer in Kammern aufgeteilten Struktur, welche Diffusion von Wasser zwischen dem wässrigen Gel und der die paramagnetische Substanz enthaltenden Kammer ermöglicht, worin i in $T_i$ 1 oder 2 ist.

**15.** Wässrige Zusammensetzung mit einem gewählten $1/T_i$-Wert, der über einen mindestens 10°C-Temperaturbereich zwischen 15 und 40°C im wesentlichen invariant ist, und umfassend ein wässriges Matrixmaterial mit einer Nicht-Null-$1/T_i$-Temperaturabhängigkeit innerhalb des Temperaturbereichs, worin darin verteilt sind (a) ein erstes paramagnetisches Material mit einer $T_i$-Relaxtivität, die innerhalb des Bereichs im wesentlichen invariant ist, und (b) ein zweites paramagnetisches Material mit einer $T_i$-Relaxivität innerhalb des Bereichs, die eine Nicht-Null-Temperaturabhängigkeit entgegengesetzter Polarität zu der Temperaturabhängigkeit von $1/T_i$ des Matrixmaterials zeigt und welche, wenn das erste und zweite Material das gleiche paramagnetische Metall enthalten, größer ist als diejenige des ersten paramagnetischen Materials, worin i in $T_i$ 1 oder 2 ist.

**16.** Zusammensetzung nach Anspruch 15, wobei das wässrige Matrixmaterial Wasser ist.

**17.** Medizinische Vorrichtung, beinhaltend eine Markerzusammensetzung wie in mindestens einem der Ansprüche 1 bis 16 definiert.

**18.** Bekleidungsgegenstand, beinhaltend eine Markerzusammensetzung wie in mindestens einem der Ansprüche 1 bis 16 definiert.

**19.** Verfahren der MR-Bilderzeugung, **dadurch gekennzeichnet, dass** das abzubildende Volumen als externen Marker eine Zusammensetzung wie in mindestens einem der Ansprüche 1 bis 16 definiert, enthält.

**20.** Verfahren nach Anspruch 19, wobei das abzubildende Subjekt ein Mensch ist und die Zusammensetzung in einem Behälter angeorndet ist, der freisetzbar mit einem Spielzeug assoziiert ist.

**21.** Verfahren zur Eichung einer MR-Vorrichtung, worin die detektierte MR-Signälstärke geeicht wird unter Verwendung einer Zusammensetzung wie in mindestens einem der Ansprüche 1 bis 16 definiert.

**22.** Verfahren zur Bestimmung der Kontrastmittelkonzentration innerhalb einer interessierenden Region in einem Patienten, welches Verfahren das Vergleichen der detektierten MR-Signalintensität aus der Region mit dem detektierten Signal von einer Markerzusammensetzung wie in mindestens einem der Ansprüche 1 bis 16 definiert, umfasst.

**23.** Verfahren nach Anspruch 22, wobei Prozentverstärkungs(%ENH)-Werte aus dem Signal-zu-Marker-Verhältnis (SMR) berechnet werden.

**Revendications**

**1.** Ensemble de compositions aqueuses de marqueur, chaque composition ayant une valeur $1/T_i$ choisie qui est essentiellement invariante sur une plage de température d'au moins 10 °C entre 15 et 40 °C et comprenant un matériau aqueux de matrice ayant une dépendance non nulle de $1/T_i$ en fonction de la température à l'intérieur de ladite plage de température, avec, réparti à l'intérieur, un premier matériau paramagnétique ayant une relaxivité $T_i$ qui est essentiellement invariante à l'intérieur de ladite plage et/ou un second matériau paramagnétique ayant à l'intérieur de ladite plage une relaxivité $T_i$ qui a une dépendance non nulle en fonction de la température, de polarité opposée à la dépendance de $1/T_i$ en fonction de la température dudit matériau de matrice et qui, si lesdits premier et second matériaux contiennent le même métal paramagnétique est supérieure à celle dudit premier matériau, ledit ensemble contenant une multiplicité desdites compositions avec différentes valeurs $1/T_i$ choisies, comprenant au moins une dite composition contenant ledit second matériau paramagnétique et au moins une dite composition contenant ledit premier matériau paramagnétique, où i dans $T_i$ est 1 ou 2.

**2.** Ensemble selon la revendication 1 dans lequel chaque composition a une dite valeur choisie $1/T_i$ qui est essentiellement invariante dans la gamme de 25 à 35 °C.

**3.** Ensemble selon l'une des revendications 1 et 2 dans lequel chaque composition a une dite valeur $1/T_i$ choisie qui est essentiellement invariante dans une gamme d'intensité de champ magnétique d'au moins ± 2 % autour d'une valeur d'intensité de champ magnétique choisie entre 0,01 et 5 T.

**4.** Ensemble selon l'une quelconque des revendications 1 à 3 contenant une multiplicité desdites compositions avec différentes valeurs $1/T_i$ choisies couvrant la gamme allant de 1,0 à 2,5 $s^{-1}$.

**5.** Ensemble selon l'une quelconque des revendications 1 à 4 contenant au moins une dite composition contenant ledit premier matériau paramagnétique et au moins une dite composition contenant ledit second matériau paramagnétique.

**6.** Ensemble selon l'une quelconque des revendications 1 à 5 contenant au moins une dite composition dans laquelle ledit matériau aqueux de matrice est l'eau.

**7.** Ensemble selon la revendication 6 dans lequel ladite eau comprend $H_2O$ et $D_2O$.

**8.** Ensemble selon l'une quelconque des revendications 1 à 7 contenant au moins une dite composition dans laquelle ledit matériau aqueux de matrice est un gel aqueux.

**9.** Ensemble selon la revendication 8 contenant au moins une dite composition dans lequel ledit gel est un polyacrylamide ou un gel d'agarose.

**10.** Ensemble selon l'une quelconque des revendications 1 à 9 dans lequel lesdites compositions sont disposées à l'intérieur de récipients fermés.

**11.** Ensemble selon la revendication 10 contenant au moins une dite composition qui est disposé dans une chambre pratiquement complètement sans espace de tête libre dans ledit récipient fermé et contient de l'eau comme ledit matériau aqueux de matrice.

**12.** Composition aqueuse ayant une valeur choisie de $1/T_i$ qui est essentiellement invariante sur une plage de température d'au moins 10 °C entre 15 et 40 °C et comprenant un gel aqueux d'un polymère hydrophile synthétique ou naturel ayant une dépendance de $1/T_i$ non nulle à l'intérieur de ladite plage de température, avec, réparti à l'intérieur, (a) un premier polychélate de gadolinium ayant une relaxivité $T_i$ qui est essentiellement invariante à l'intérieur de ladite plage et/ou (b) un second polychélate de gadolinium ayant à l'intérieur de ladite plage une relaxivité $T_i$ qui est supérieure à celle dudit premier polychélate et qui a une dépendance non nulle en fonction de la température, de polarité opposée à la dépendance en fonction de la température de $1/T_i$ dudit gel aqueux, où i dans $T_i$ est 1 ou 2.

**13.** Composition aqueuse de gel selon la revendication 12 comprenant un polymère hydrophile formant un gel et un chélate de gadolinium d'un polychélatant ayant des motifs répétés de formule

$$\textbf{[-Ch-L-]}_n$$

(dans laquelle Ch est un fragment chélatant, L est un fragment lieur organique hydrophile ou hydrophobe et n est un entier).

**14.** Composition aqueuse ayant une valeur choisie de $1/T_i$ qui est essentiellement invariante sur une plage de température d'au moins 10 °C entre 15 et 40 °C comprenant un gel aqueux d'un polymère hydrophile synthétique ou naturel ayant une dépendance de $1/T_i$ non nulle à l'intérieur de ladite plage de température, avec, réparti à l'intérieur, un matériau contenant de l'eau et une substance paramagnétique dans une structure compartimentée permettant la diffusion d'eau entre le gel aqueux et le compartiment contenant ladite substance paramagnétique, où i dans $T_i$ est 1 ou 2.

**15.** Composition aqueuse ayant une valeur choisie de $1/T_i$ qui est essentiellement invariante sur une plage de température d'au moins 10 °C entre 15 et 40 °C comprenant un matériau aqueux de matrice ayant une dépendance de $1/T_i$ non nulle en fonction de la température à l'intérieur de ladite plage de température, avec, réparti à l'intérieur, (a) un premier matériau paramagnétique ayant une relaxivité $T_i$ qui est essentiellement invariante à l'intérieur de ladite plage et (b) un second matériau paramagnétique ayant à l'intérieur de ladite plage une relaxivité $T_i$ qui a une dépendance non nulle en fonction de la température, de polarité opposée à la dépendance en fonction de la température de $1/T_i$ dudit matériau de matrice et qui, si lesdits premier et second matériaux contiennent le même métal paramagnétique est supérieure à celle du premier matériau paramagnétique, où i dans $T_i$ est 1 ou 2.

**16.** Composition selon la revendication 15 dans laquelle ledit matériau aqueux de matrice est l'eau.

**17.** Dispositif médical incorporant une composition de marqueur telle que définie dans l'une quelconque des revendications 1 à 16.

**18.** Article d'habillement incorporant une composition de marqueur selon l'une quelconque des revendications 1 à 16.

**19.** Méthode d'imagerie RM **caractérisée en ce que** le volume dont on forme l'image contient comme marqueur externe une composition selon l'une quelconque des revendications 1 à 16.

**20.** Méthode selon la revendication 19 dans laquelle le sujet dont on forme l'image est un humain et ladite composition est disposée dans un récipient associé de manière amovible à un jouet.

**21.** Méthode de calibrage d'un appareil RM dans lequel l'intensité de signal RM détectée est calibrée en utilisant une composition telle que définie dans l'une quelconque des revendications 1 à 16.

**22.** Méthode d'estimation de la concentration d'un agent de contraste à l'intérieur d'une région d'intérêt chez un patient, laquelle méthode comprend la comparaison de l'intensité de signal RM détecté provenant de ladite région avec le signal détecté provenant d'une composition de marqueur telle que définie dans l'une quelconque des revendications 1 à 16.

**23.** Méthode selon la revendication 22 dans laquelle les valeurs de pourcentage d'accroissement (%ENH, percent enhancement) sont calculées à partir du rapport signal sur marqueur (SMR, signal-to-marker ratio).

FIG. 1

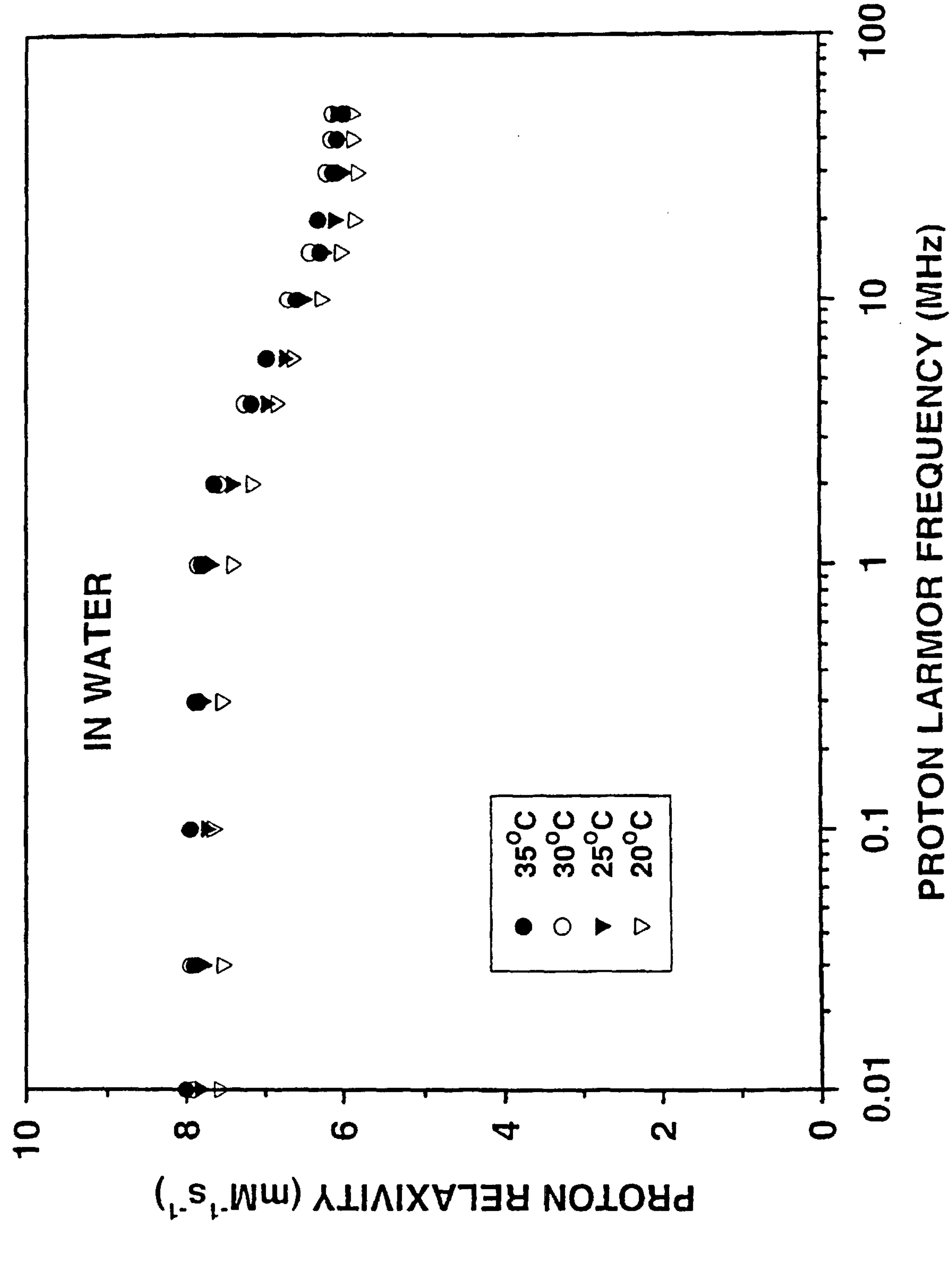
IN WATER
35°C
30°C
25°C
20°C
PROTON LARMOR FREQUENCY (MHz)
PROTON RELAXIVITY (mM⁻¹s⁻¹)
0.01
0.1
1
10
100
0
2
4
6
8
10

FIG. 2

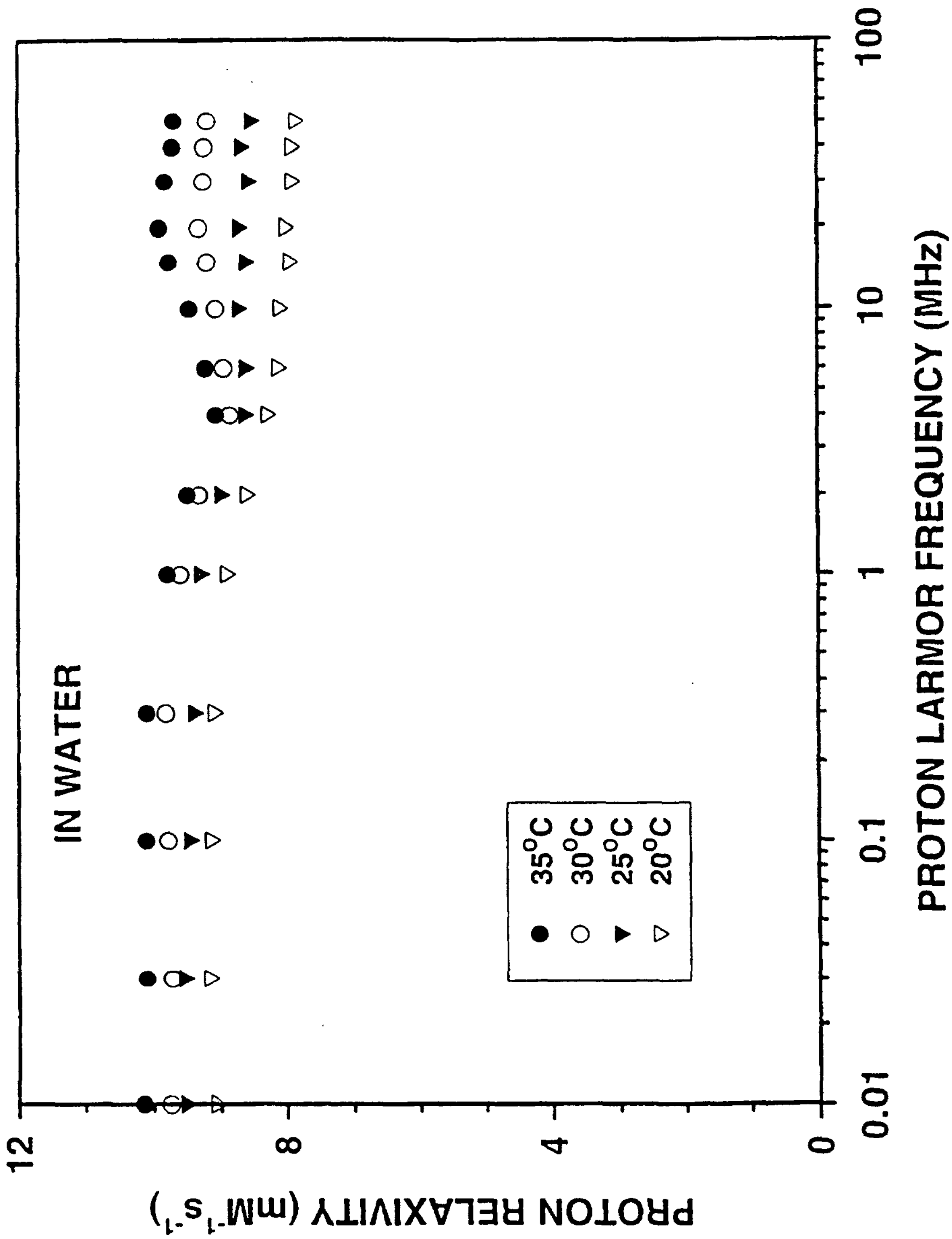
IN WATER
35°C
30°C
25°C
20°C
PROTON RELAXIVITY ($mM^{-1}s^{-1}$)
PROTON LARMOR FREQUENCY (MHz)
0
4
8
12
0.01
0.1
1
10
100

FIG. 3

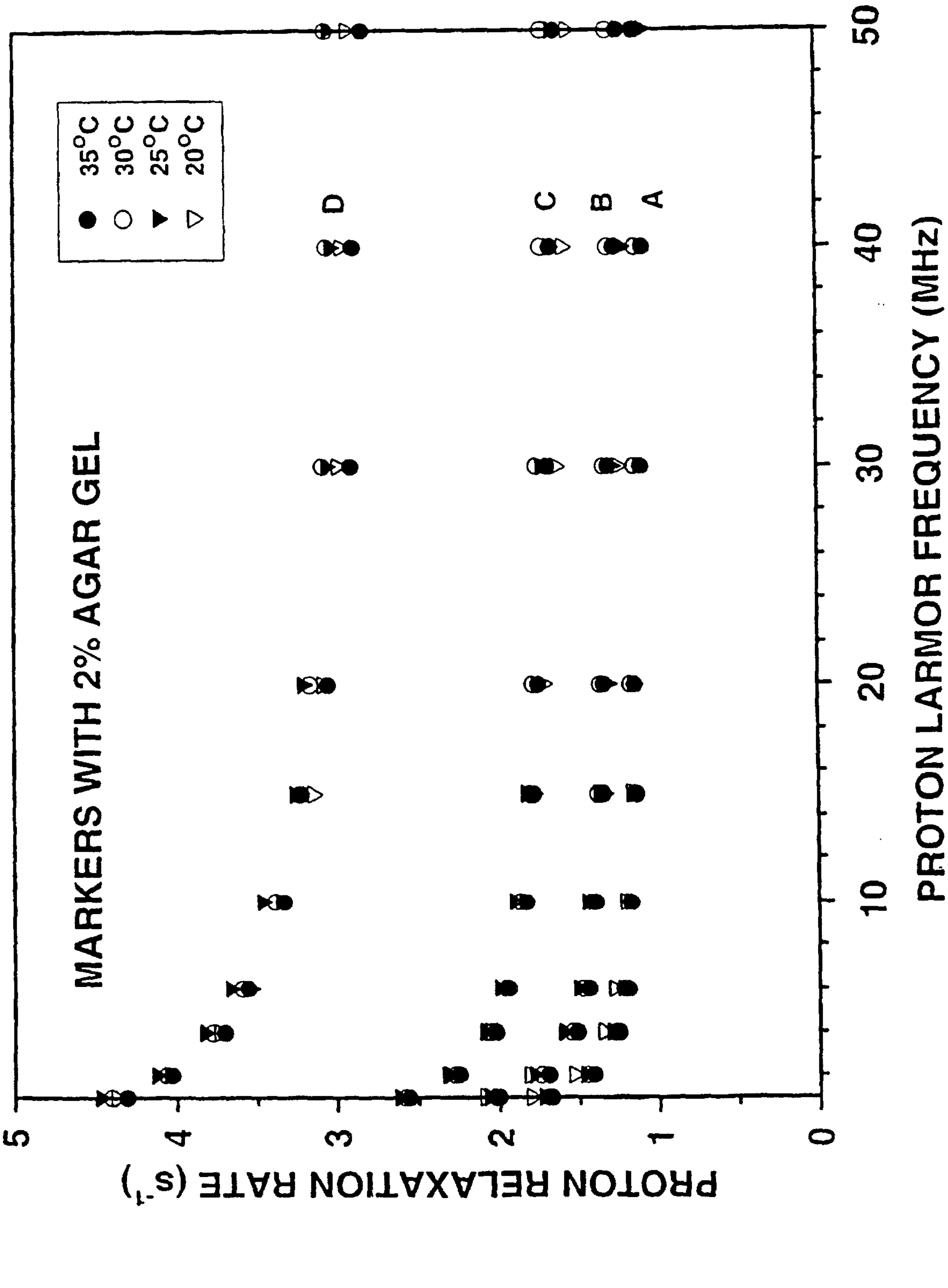
MARKERS WITH 2% AGAR GEL
35°C
30°C
25°C
20°C
D
C
B
A
PROTON RELAXATION RATE (s-1)
PROTON LARMOR FREQUENCY (MHz)
0
1
2
3
4
5
10
20
30
40
50

FIG. 4

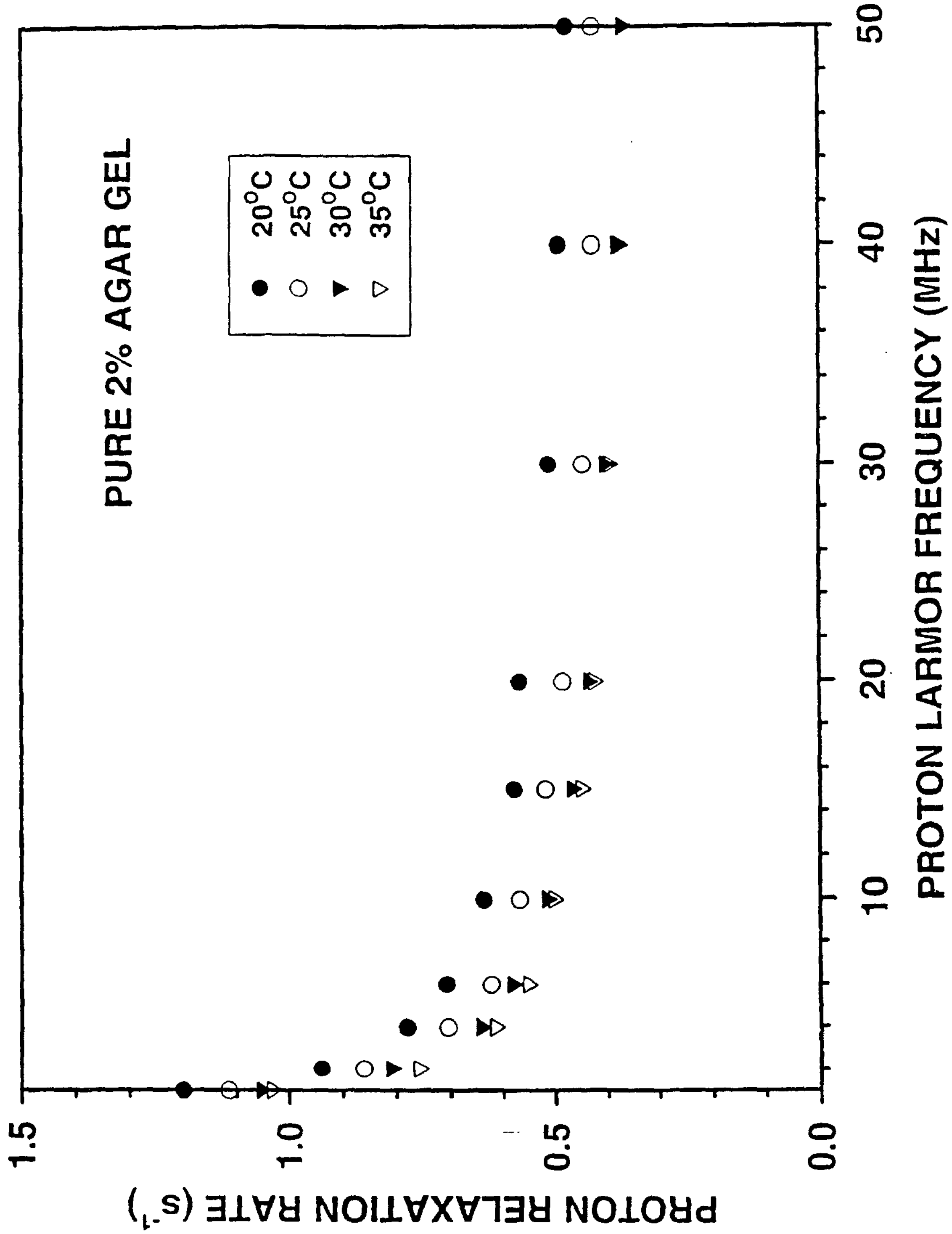
PURE 2% AGAR GEL
20°C
25°C
30°C
35°C
PROTON LARMOR FREQUENCY (MHz)
10
20
30
40
50
PROTON RELAXATION RATE ($s^{-1}$)
0.0
0.5
1.0
1.5

FIG. 5

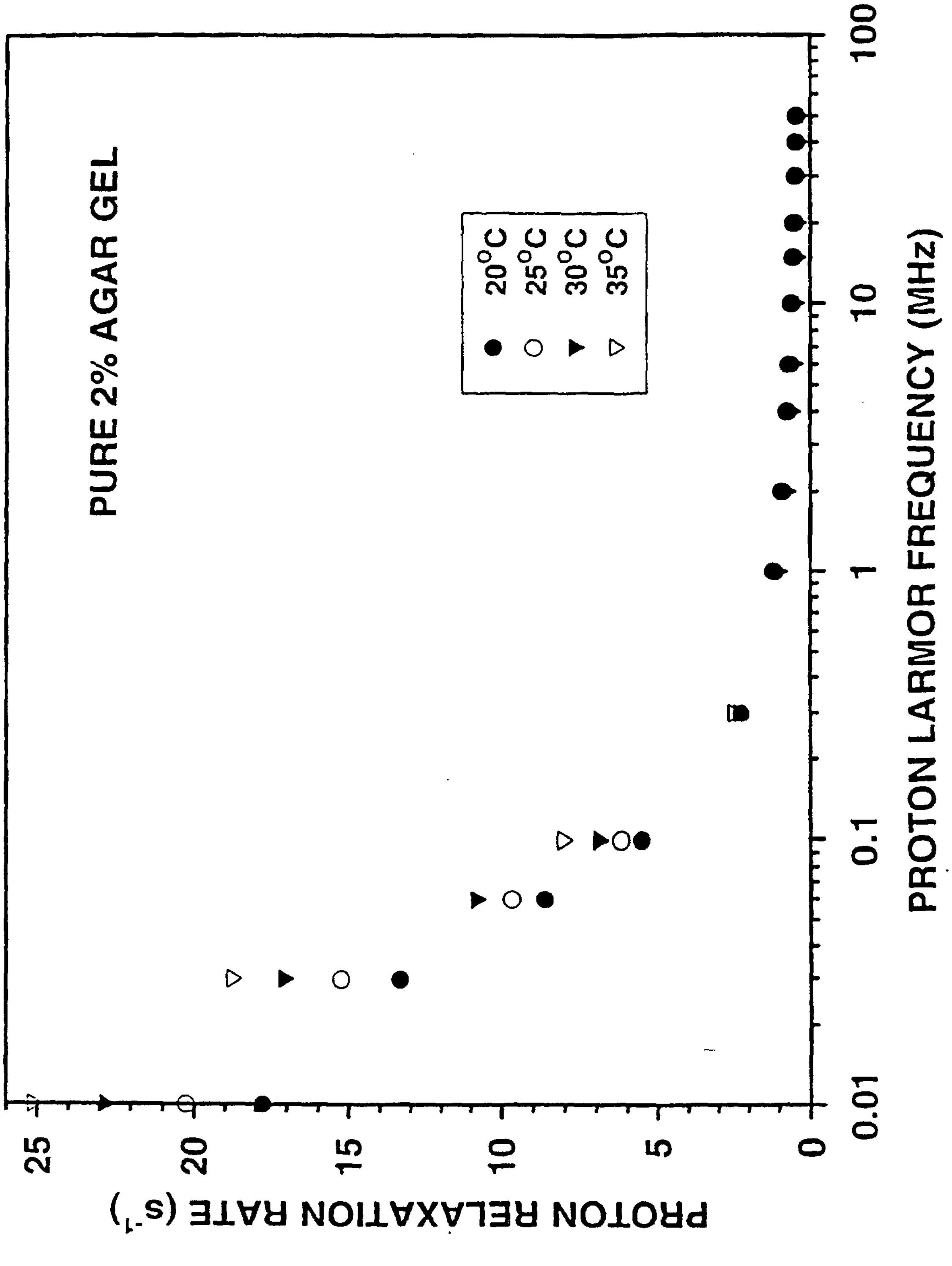
PURE 2% AGAR GEL
20°C
25°C
30°C
35°C
0.01
0.1
1
10
100
PROTON LARMOR FREQUENCY (MHz)
0
5
10
15
20
25
PROTON RELAXATION RATE (s-1)

FIG. 6

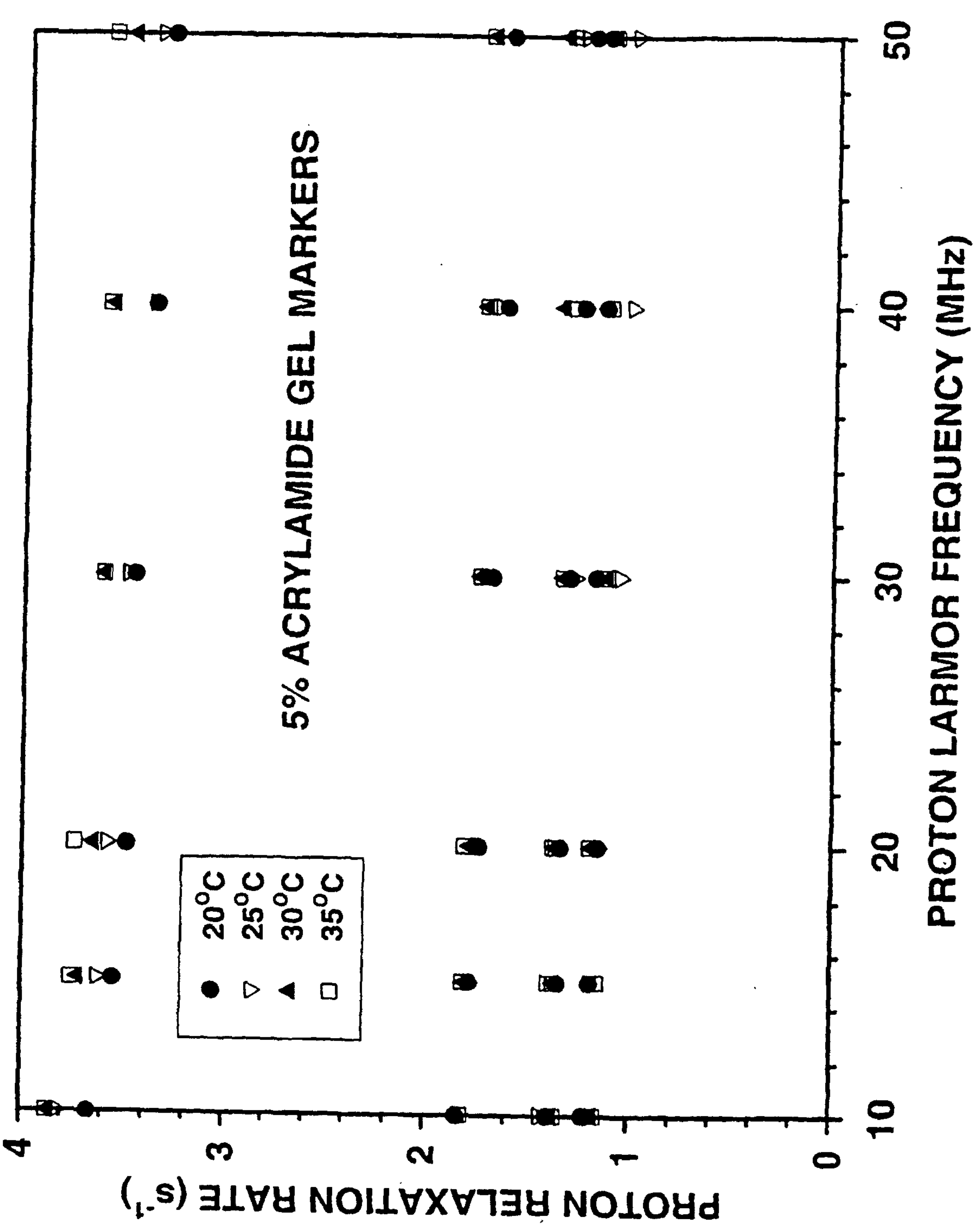

5% ACRYLAMIDE GEL MARKERS
20°C
25°C
30°C
35°C
PROTON LARMOR FREQUENCY (MHz)
PROTON RELAXATION RATE (s-1)
10
20
30
40
50
0
1
2
3
4

FIG. 7

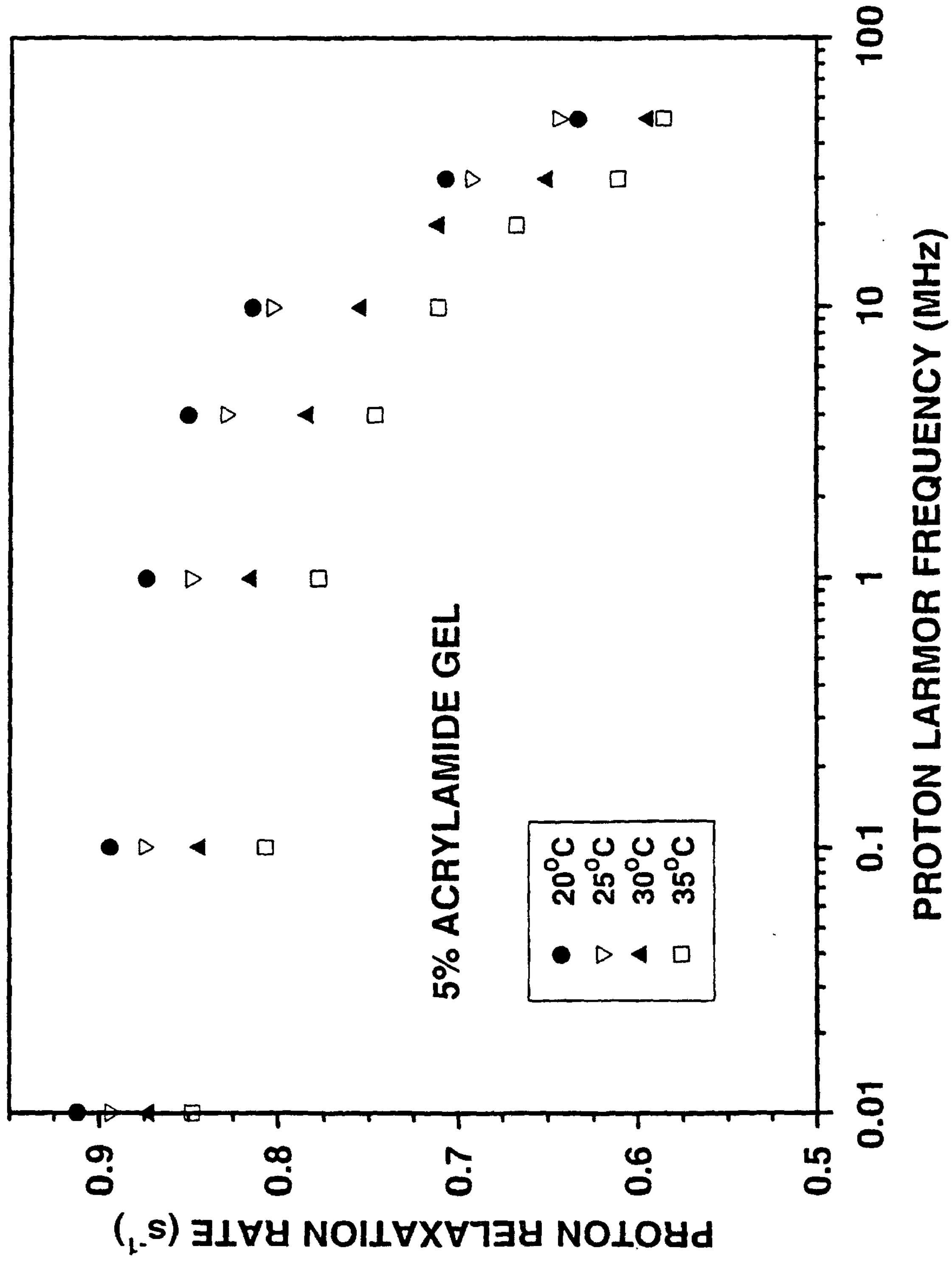
5% ACRYLAMIDE GEL
● 20°C
▷ 25°C
▲ 30°C
□ 35°C
PROTON RELAXATION RATE (s-1)
0.9
0.8
0.7
0.6
0.5
0.01
0.1
1
10
100
PROTON LARMOR FREQUENCY (MHz)

FIG. 8

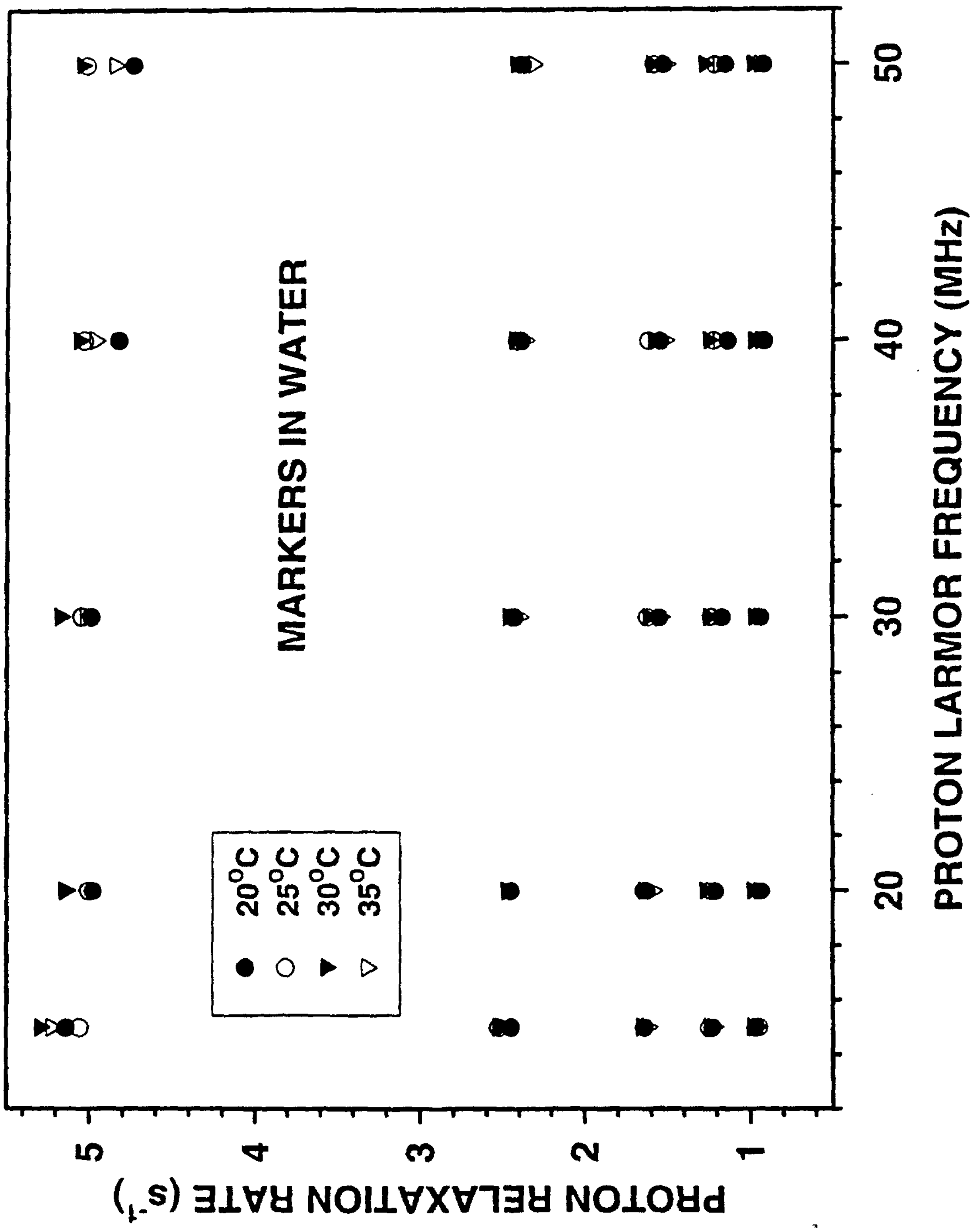
MARKERS IN WATER
20°C
25°C
30°C
35°C
PROTON LARMOR FREQUENCY (MHz)
20
30
40
50
PROTON RELAXATION RATE ($s^{-1}$)
1
2
3
4
5

FIG. 8A

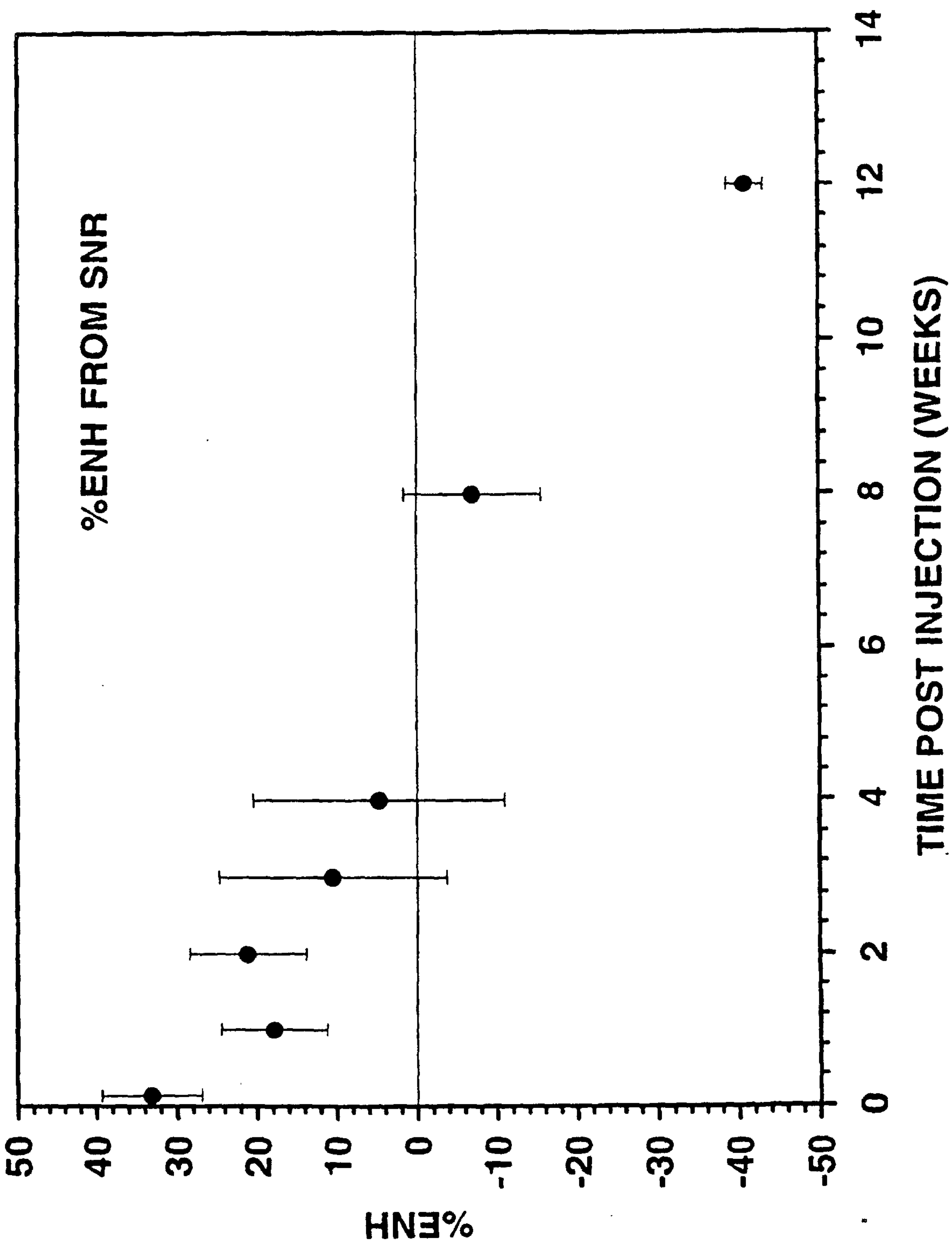

%ENH FROM SNR
%ENH
50
40
30
20
10
0
-10
-20
-30
-40
-50
0
2
4
6
8
10
12
14
TIME POST INJECTION (WEEKS)

FIG. 8B

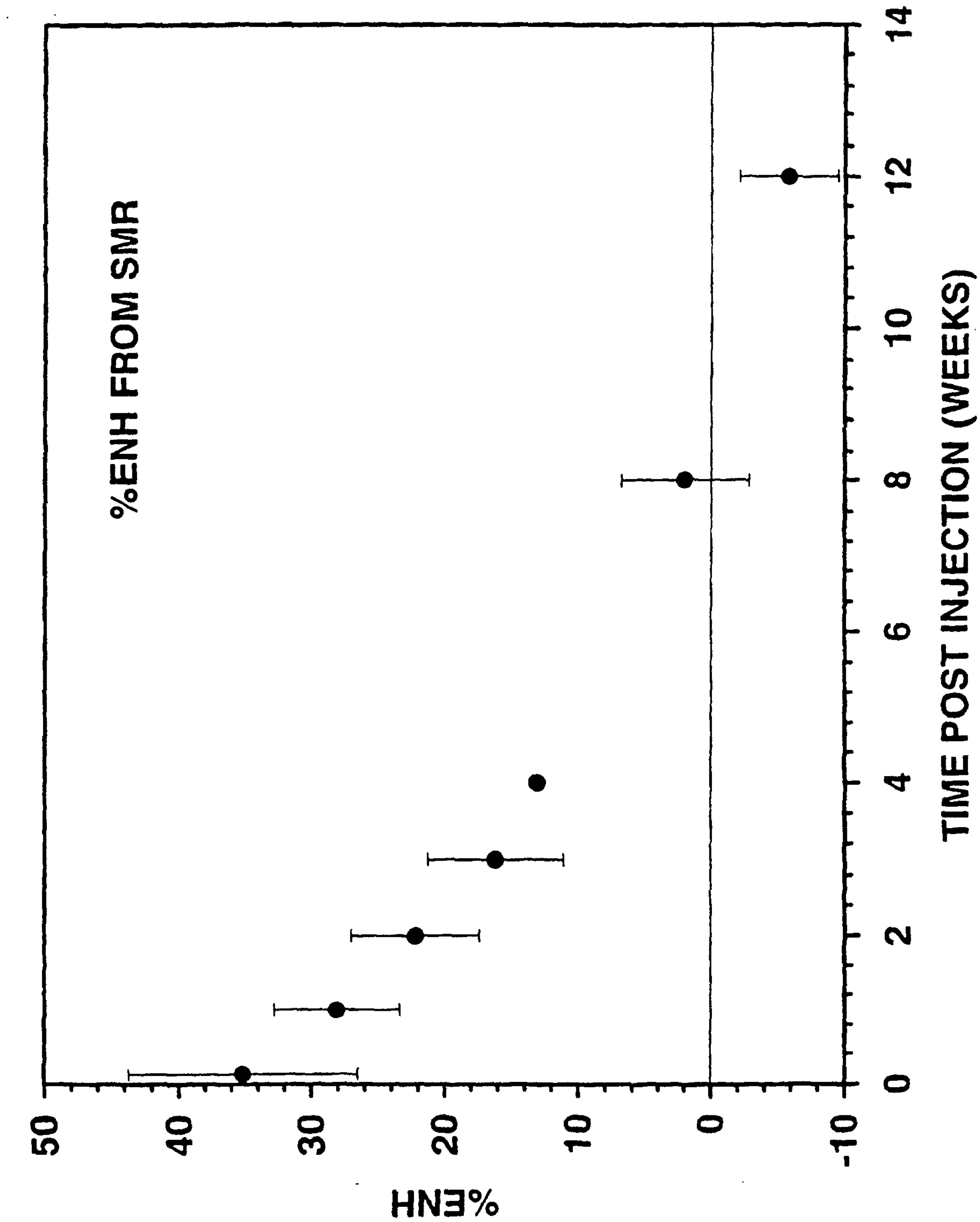
%ENH FROM SMR
%ENH
50
40
30
20
10
0
-10
0
2
4
6
8
10
12
14
TIME POST INJECTION (WEEKS)

FIG. 9

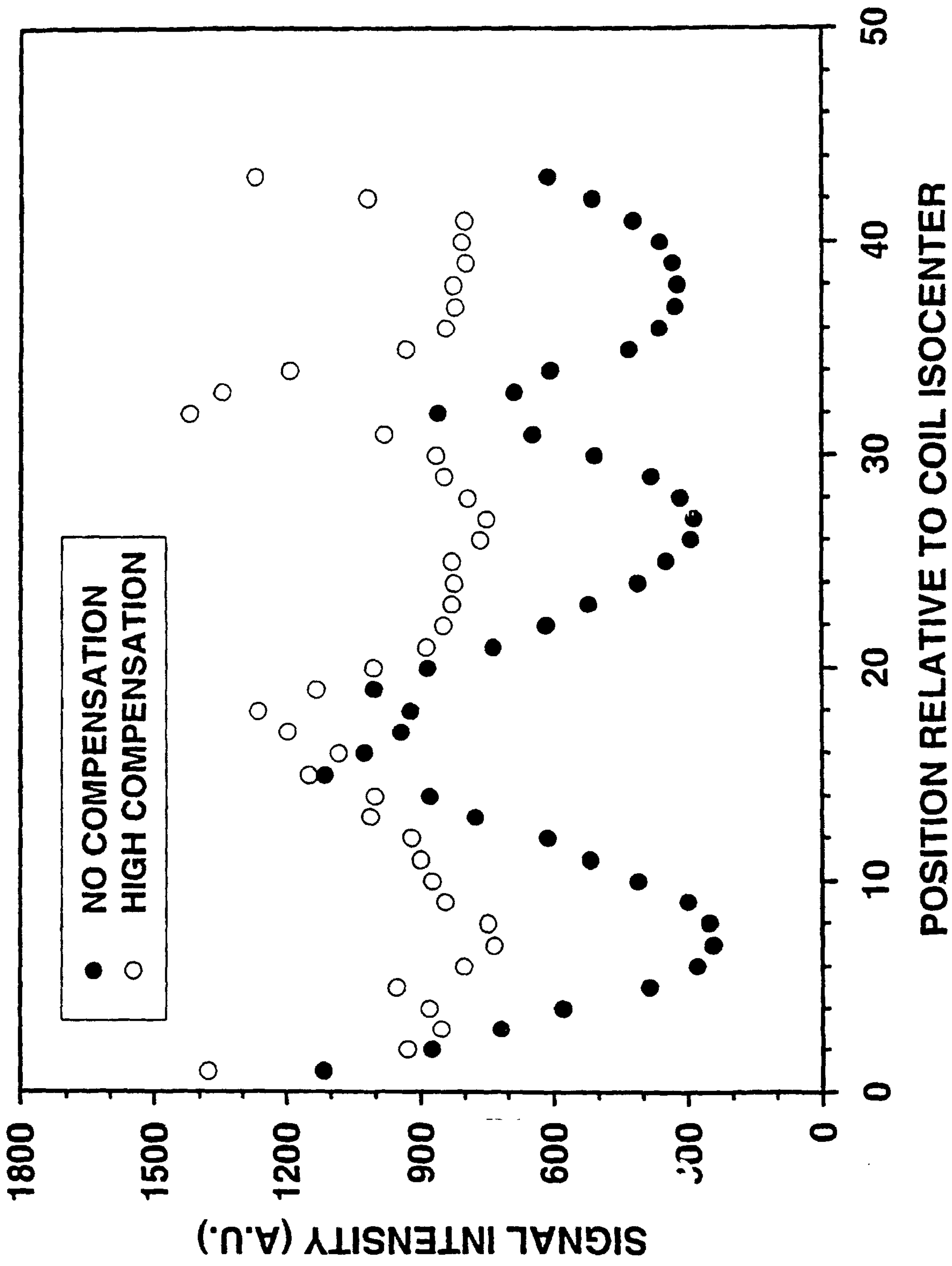
NO COMPENSATION
HIGH COMPENSATION
SIGNAL INTENSITY (A.U.)
1800
1500
1200
900
600
300
0
POSITION RELATIVE TO COIL ISOCENTER
0
10
20
30
40
50

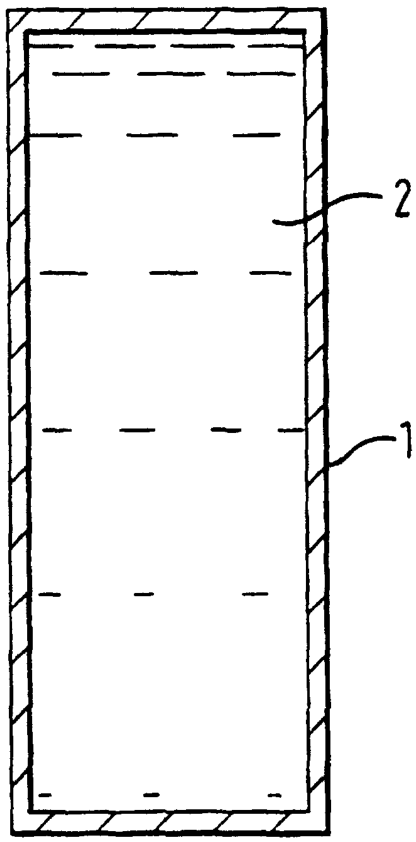


FIG. 10